# EUROPEAN PATENT SPECIFICATION

(11) **EP 2 880 180 B1**
(45) Date of publication and mention of the grant of the patent: **03.10.2018**
(21) Application number: 13745117.5
(22) Date of filing: 05.08.2013
(51) Int. Cl.: C12Q 1/6886

(54) **METHODS AND KITS FOR SCREENING PATIENTS WITH A CANCER**
VERFAHREN UND KITS ZUM SCREENING VON PATIENTEN MIT KREBS
PROCÉDÉS ET KITS DE CRIBLAGE DE PATIENTS ATTEINTS D'UN CANCER

(30) Priority: 06.08.2012 EP 12305975
(43) Date of publication of application: 10.06.2015
(73) Proprietor: Institut National de la Santé et de la Recherche Médicale (INSERM), 75013 Paris (FR); Université Paris Descartes, 75006 Paris (FR); Assistance Publique Hôpitaux De Paris, 75004 Paris 4 (FR)
(72) Inventor: GALON, Jérome, F-75006 Paris (FR); PAGES, Franck, F-75006 Paris (FR); MLECNIK, Bernhard, F-75006 Paris (FR); BINDEA, Gabriela, F-75006 Paris (FR)
(74) Representative: Cabinet Plasseraud
(86) International application number: PCT/EP2013/066425
(87) International publication number: WO 2014/023706

(56) References cited:
- EP-A1- 1 777 523
- WO-A1-2012/095448
- WO-A2-2005/024043
- WO-A2-2011/153325
- WO-A2-2012/038068
- FRANCK PAGÉS ET AL: "Prognostic impact of anticancer immune responses: an introduction", SEMINARS IN IMMUNOPATHOLOGY, vol. 33, no. 4, 1 July 2011 (2011-07-01), pages 317-319, XP055042377, ISSN: 1863-2297, DOI: 10.1007/s00281-011-0278-4
- KRISTOFFER WATTEN BRUDVIK ET AL: "Regulatory T-cell-mediated inhibition of antitumor immune responses is associated with clinical outcome in patients with liver metastasis from colorectal cancer", CANCER IMMUNOLOGY, IMMUNOTHERAPY, SPRINGER, BERLIN, DE, vol. 61, no. 7, 11 December 2011 (2011-12-11), pages 1045-1053, XP035074367, ISSN: 1432-0851, DOI: 10.1007/S00262-011-1174-4
- F PAGÈS ET AL: "Immune infiltration in human tumors: a prognostic factor that should not be ignored", ONCOGENE, vol. 29, no. 8, 25 February 2010 (2010-02-25), pages 1093-1102, XP055025840, ISSN: 0950-9232, DOI: 10.1038/onc.2009.416
- VANESSA DESCHOOLMEESTER ET AL: "Immune Cells in Colorectal Cancer: Prognostic Relevance and Role of MSI", CANCER MICROENVIRONMENT ; OFFICIAL JOURNAL OF THE INTERNATIONAL CANCER MICROENVIRONMENT SOCIETY, SPRINGER NETHERLANDS, DORDRECHT, vol. 4, no. 3, 27 May 2011 (2011-05-27), pages 377-392, XP019988885, ISSN: 1875-2284, DOI: 10.1007/S12307-011-0068-5

## Description

### FIELD OF THE INVENTION:

The present invention relates to methods and kits for screening patients with a cancer, particularly for determining whether a patient with a cancer would benefit of an antitumoral treatment.

### BACKGROUND OF THE INVENTION:

Today, cancers are generally classified according to the UICC-TNM system. The TNM (for "Tumor-Node-Metastasis") staging system uses the size of the tumor, the presence or absence of tumor in regional lymph nodes, and the presence or absence of distant metastases, to assign a stage and an outcome to the tumor.

The TNM system developed from the observation that patients with small tumours have better prognosis than those with tumours of greater size at the primary site. In general, patients with tumours confined to the primary site have better prognosis than those with regional lymph node involvement, which in turn is better than for those with distant spread of disease from one body part two another. Accordingly, cancers are usually staged into four levels. Stage I cancer is very localized cancer with no cancer in the lymph nodes. Stage II cancer has spread near to where the cancer started. Stage III cancer has spread to lymph nodes. Stage IV cancer has spread to a distant part of the body. The assigned stage is used as a basis for selection of appropriate therapy and for prognostic purposes. For example chemotherapy is always recommended for patients with stage IV cancers. On the contrary, there are no relevant guidelines for prescribing chemotherapy for patient with a UICC-TNM stage I or II cancer. Accordingly there is a need for reliable diagnostic tools to guide treatment decisions is all the more as an essential step for the multitude of available new therapies is the efficient selection of patients for adequate cancer therapy.

In humans, regulation of gene expression refers to the control of the amount and timing of appearance of the functional product of a gene. Control of gene expression is vital to allow a cell to produce gene products when needed. The way that the information in genes is turned into gene products is regulated. In a short summary, regulation consists in a balance between actions of activators and inhibitors.

EP 1 777 523 discloses methods for determining the outcome of a cancer in a patient, which are based on the quantification of one or several biological markers that are indicative of the presence of, or alternatively the level of, the adaptive immune response of said patient against said cancer.

WO2005/024043 discloses the use of Affimetrix U133A and U133B microarrays which comprise genes of the immune adaptive and immunosuppressive responses.

WO2012/038068 discloses the prediction of efficacy of a treatment in cancer patients by quantifying markers of the adaptive and innate immune response of said patient against cancer.

Pages et al (Seminars in Immunopathology; vol. 33, no. 4, pp. 317-319, 2011), Kristoffer Watten Brudvik et al (Cancer in Immunology, Immunotherapy, Vol. 61, no. 7, pp. 1045-1053, 2011) and Pages et al (Oncogene, vol. 29, no. 8, pp. 1093-1102, 2010) discuss the relationship between cancer and the immune system.

Deschoomeester et al (Cancer Microenvironment; Official Journal of the International Cancer Microenvironment Society, vol. 4, no. 3, pp. 377-392, 2011) discuss the prognostic relevance and role of microsatellite instability in colorectal cancer.

After extensive researches, for patients without distant metastasis (Stage IV), the inventors have found :
1. That a patient with low expression levels for the genes of the immune adaptive response and high expression levels for the genes representative of the immunosupressive response not only will have a bad prognosis (e.g. a short disease-free survival time) but also will not significantly improve his survival in case of treatment.
2. that a patient with high expression levels for the genes of the immune adaptive response and low expression levels for the genes representative of the immunosupressive response will not only have a good prognosis (e.g. a long disease-free survival time) but also will not significantly improve his survival in case of treatment.
3. that a patient with high expression levels for the genes of the immune adaptive response and high expression levels for the genes representative of the immunosupressive response will show an intermediate prognosis, but an antitumoral treatment will a significant impact on his survival (e.g from an intermediate prognosis to a good prognosis).

The two first groups of patients are to be considered as "bad responders" (i.e. the treatment will have a limited (or moderate) impact on their survival), whereas the third group of patients is to be considered as "good responders".

### SUMMARY OF THE INVENTION:

The invention is defined by the claims.

A subject of the present invention is therefore a method and kits for screening patients with a cancer, particularly for determining whether a patient with a cancer would benefit of an antitumoral treatment.

### DETAILED DESCRIPTION OF THE INVENTION:

The present invention relates to a method for assessing the responsiveness of a patient suffering from solid cancer to anticancer treatment omprising
i) determining in a tumor sample obtained from a patient an expression level of at least one gene representative of human adaptive immune response and an expression level of at least one gene representative of human immunosuppressive response,
ii) comparing the expression levels of said at least one gene representative of human adaptive immune response and said at least one gene representative of human immunosuppressive response determined at step i) with predetermined reference values selected such as said predetermined reference values separate a panel of patients with a cancer into two groupings according to the expression level of said genes and to survival of patients according to Kaplan Meier curves analyses and associated logrank p values,
iii) concluding whether the patient has a good (level higher than the predetermined reference value) or a bad (level lower than the predetermined reference value) adaptive immune response and a good or a bad immunosuppressive response; and
iv) determining whether the patient will advantageously receive an antitumoral treatment (responder to antitumaroal treatment) in view of said good or bad adaptative immune and immunosuppressive responses.

A patient who has a good adaptive immune response and a good immunosuppressive response could benefit of an anti tumoral treatment.

In one embodiment of the invention, the patient subjected to the above method suffers from a solid cancer selected from the group consisting of adrenal cortical cancer, anal cancer, bile duct cancer (e.g. periphilar cancer, distal bile duct cancer, intrahepatic bile duct cancer), bladder cancer, bone cancer (e.g. osteoblastoma, osteochrondroma, hemangioma, chondromyxoid fibroma, osteosarcoma, chondrosarcoma, fibrosarcoma, malignant fibrous histiocytoma, giant cell tumor of the bone, chordoma), brain and central nervous system cancer (e.g. meningioma, astocytoma, oligodendrogliomas, ependymoma, gliomas, medulloblastoma, ganglioglioma, Schwannoma, germinoma, craniopharyngioma), breast cancer (e.g. ductal carcinoma in situ, infiltrating ductal carcinoma, infiltrating lobular carcinoma, lobular carcinoma in situ, gynecomastia), Castleman disease (e.g. giant lymph node hyperplasia, angiofollicular lymph node hyperplasia), cervical cancer, colorectal cancer, endometrial cancer (e.g. endometrial adenocarcinoma, adenocanthoma, papillary serous adnocarcinoma, clear cell), esophagus cancer, gallbladder cancer (mucinous adenocarcinoma, small cell carcinoma), gastrointestinal carcinoid tumors (e.g. choriocarcinoma, chorioadenoma destruens), Hodgkin's disease, Kaposi's sarcoma, kidney cancer (e.g. renal cell cancer), laryngeal and hypopharyngeal cancer, liver cancer (e.g. hemangioma, hepatic adenoma, focal nodular hyperplasia, hepatocellular carcinoma), lung cancer (e.g. small cell lung cancer, non-small cell lung cancer), mesothelioma, plasmacytoma, nasal cavity and paranasal sinus cancer (e.g. esthesioneuroblastoma, midline granuloma), nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma (e.g. embryonal rhabdomyosarcoma, alveolar rhabdomyosarcoma, pleomorphic rhabdomyosarcoma), salivary gland cancer, skin cancer (e.g. melanoma, nonmelanoma skin cancer), stomach cancer, testicular cancer (e.g. seminoma, nonseminoma germ cell cancer), thymus cancer, thyroid cancer (e.g. follicular carcinoma, anaplastic carcinoma, poorly differentiated carcinoma, medullary thyroid carcinoma,), vaginal cancer, vulvar cancer, and uterine cancer (e.g. uterine leiomyosarcoma).

The term "tumor sample" means any tissue sample derived from the tumor of the patient. The tissue sample is obtained for the purpose of the in vitro evaluation. The sample can be fresh, frozen, fixed (e.g., formalin fixed), or embedded (e.g., paraffin embedded). In a particular embodiment the sample results from biopsy performed in a tumour sample of the patient. An example is an endoscopical biopsy performed in the bowel of the patient suffering from colorectal cancer.

Under preferred conditions of implementation of the invention, an expression level EL₁ of a single gene representative of human adaptive immune response and of a single gene representative of human immunosuppressive response (a pair of genes) is assessed in the method of the invention. Preferably one to three genes of each and more preferably one or two genes of each are used. A limited number of genes of each kind provides good and reliable results and is easy to implement. Particularly a single reference value is sufficient for each of both genes. The higher the number of genes, the more sophisticated is the reference value. Examples of determination of reference values are given thereafter.

The use of more genes than one or two pairs of genes is more difficult to implement and more expensive and time consuming but however provides other advantages. For example if the assessment of the expression level of one gene is erroneous, the overall result is compensated by the reserved of the other genes of the same kind (human adaptive immune response or immunosuppressive response).

As used herein the expression "gene representative of the adaptive immune response" refers to any gene that is expressed by a cell that is an actor of the adaptive immune response in the tumor or that contributes to the settlement of the adaptive immune response in the tumor. The adaptive immune response, also called "acquired immune response", comprises antigen-dependent stimulation of T cell subtypes, B cell activation and antibody production,. For example cells of the adapative immune response include but are not limited to cytotoxic T cells, T memory T cells, Th1 and Th2 cells, activated macrophages and activated dendritic cells, NK cells and NKT cells. Accordingly, a gene representative of the adaptive immune response may be typically selected from the cluster of the co-modulated genes for the Th1 adaptive immunity, for the cytotoxic response, or for the memory response, and may encode for a Th1 cell surface marker, an interleukin (or an interleukin receptor), or a chemokine or (a chemokine receptor).

Genes representative of the adaptative immune response are
- the family of chemokines and chemokine receptors consisting of: CXCL13, CXCL9, CCL5, CCR2, CXCL10, CXCL11, CXCR3, CCL2 and CX3CL1,
- the family of cytokines consisting of: IL15,
- the TH1 family consisting of: IFNG, IRF1, STAT1, STAT4 and TBX21
- the family of lymphocytes membrane receptors consisting of: ITGAE, CD3D, CD3E, CD3G, CD8A, CD247, CD69 and ICOS,
- the family of cytotoxic molecules consisting of: GNLY, GZMH, GZMA, GZMB, GZMK, GZMM and PRF1,
and the kinase LTK.

The genes representative of the adaptative immune response provide the best results for the response of a patient to the treatment as shown hereafter in table 5, are reported in Table 1:

**Table 1**

| |
|---|
| CCL5 |
| CCR2 |
| CD247 |
| CD3E |
| CD3G |
| CD8A |
| CX3CL1 |
| CXCL11 |
| GZMA |
| GZMB |
| GZMH |
| GZMK |
| IFNG |
| IL15 |
| IRF1 |
| ITGAE |
| PRF1 |
| STAT1 |
| TBX21 |

As used herein the expression "gene representative of the immunosuppressive response" refers to any gene that is expressed by a cell that is an actor of the immunosuppressive response in the tumor or that contributes to the settlement of the immunosuppressive response in the tumor. For example, the immunosuppressive response comprises
- co-inhibition of antigen-dependent stimulation of T cell subtypes: genes CD276, CTLA4, PDCD1, CD274, or VTCN1 (B7H4),
- inactivation of macrophages and dendritic cells and inactivation of NK cells: genes TSLP, CD1A, or VEGFA
- expression of cancer stem cell marker, differentiation and/or oncogenesis: PROM1, IHH.
- expression of immunosuppressive proteins produced in the tumour environment: genes PF4, REN, VEGFA.

For example cells of the immunosuppressive response include immature dendritic cells (CD1A), regulatory T cells (Treg cells) and Th17 cells expressing IL17A gene.

Accordingly, a gene representative of the adaptive immune response may be typically selected from the group of the co-modulated adaptive immune genes, whereas the immunosuppressive genes, may be representative of the inactivation of immune cells (e.g. dendritic cells) and may contribute to induction of an immunosuppressive response.

The gene representative of the immunosuppressive response is selected from the group consisting of genes reported in Table 2 hereunder:

**Table 2**

| |
|---|
| CD274 |
| CTLA4 |
| IHH |
| IL17A |
| PDCD1 |
| PF4 |
| PROM1 |
| REN |
| TSLP |
| VEGFA |

Said genes provide the best results for the response of a patient to the treatment as shown hereafter in table 5.

According to the invention, the expression levels of a gene representative of the adaptative immune response selected from the group consisting of GNLY, CXCL13, CX3CL1, CXCL9, ITGAE, CCL5, GZMH, IFNG, CCR2, CD3D, CD3E, CD3G, CD8A, CXCL10, CXCL11, GZMA, GZMB, GZMK, GZMM, IL15, IRF1, LTK, PRF1, STAT1, CD69, CD247, ICOS, CXCR3, STAT4, CCL2 and TBX21 and of a gene representative of the immunosuppressive response selected from the group consisting of PF4, REN, VEGFA, TSLP, IL17A, PROM1, IHH, CD1A, CTLA4, PDCD1, CD276, CD274, and VTCN1 (B7H4) are measured.

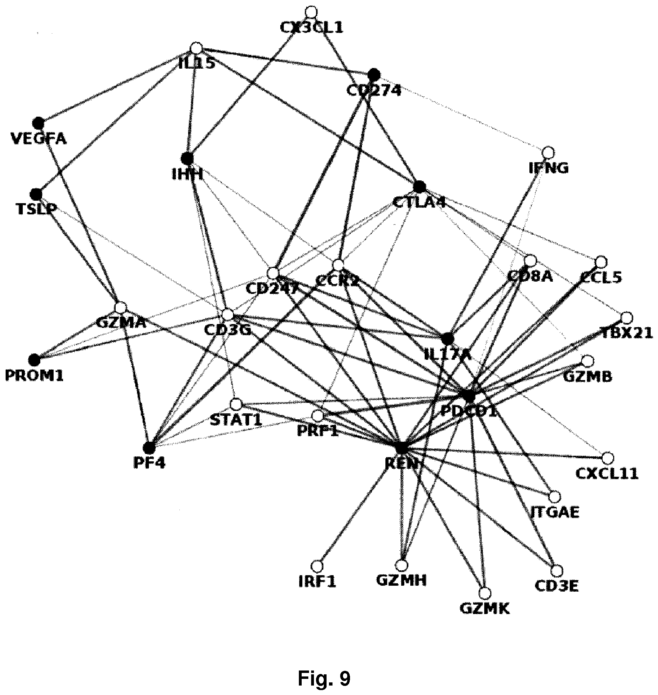

Because somes genes are more frequently found significant when combining one adaptive gene and one immunosuppressive gene (as illustrated in Figure 9), the most preferred genes are:
- genes representative of the adaptative immune response: CD3G, CD8A, CCR2 and GZMA
- genes representative of the immunosuppressive response: REN, IL17A, CTLA4 and PDCD1.

According to the present invention, a gene representative of the adaptative immune response and a gene representative of the immunosuppressive response are selected respectively from the groups consisting of the genes of Tables 1 and 2 above.

Under still preferred conditions for implementing the invention, a pair of genes is selected from the combinations of genes of table 5 hereafter. Combinations of genes of both types of figure 9 linked by thick lines are more preferred.

Combinations Nr 2, 3, 7, 8, 9, 10, 15, 41, 43, 44, 55, 56, and 66 of table 5 hereafter are preferred. Other preferred pairs of genes are combinations Nr 2, 3, 7, 8, 9, 10, 15, 41, 43, 44, 55, 56, and 66 of table 5 hereafter and CD3G-VEGF, CD3E-VEGF and CD8A-VEGF.

Preferred combinations of two pairs of genes (total of 4 genes) are
- CCR2, CD3G, IL17A and REN and
- CD8A, CCR2, REN and PDCD1.

The precise choice of the genes selected for use in the present process may depend on the type of treatment contemplated for the patient. For example, genes selected from the group consisting of CX3CL1 IL15, CD247, CD3G, CD8A, PRF1, CCL5 and TBX21 for the immunosuppressive response, preferably CX3CL1 and IL15 and gene CTLA4 for the adaptative immune response will be preferred when a treatment using a drug such as a monoclonal antibody working by activating the immune system such as Ipilimumab, also known as MDX-010 or MDX-101, marketed as Yervoy®, is contemplated for a patient.

Genes selected from the group consisting of IL15 and GZMA for the adaptative immune response, and gene VEGFA for the immunosuppressive response will be preferred when a treatment such as an antibody that inhibits vascular endothelial growth factor A (VEGF-A) such as bevacizumab marketed as Avastin®, is contemplated for a patient.

Similar considerations apply for example for the pair of genes GZMA - PDCD1 (also designated as CD279), when a treatment such as an antibody that targets PD-1 such as BMS-936558, is contemplated for a patient.

In the present specification, the name of each of the genes of interest refers to the internationally recognised name of the corresponding gene, as found in internationally recognised gene sequences and protein sequences databases, including the database from the HUGO Gene Nomenclature Committee that is available notably at the following Internet address: http://www.gene.ucl.ac.uk/nomenclature/index.html. In the present specification, the name of each of the genes of interest may also refer to the internationally recognised name of the corresponding gene, as found in the internationally recognised gene sequences database Genbank. Through these internationally recognised sequence databases, the nucleic acid to each of the gene of interest described herein may be retrieved by one skilled in the art.

The cancer prognosis method of the invention may be performed with a combination of genes provided that the combination comprises at least one one gene representative of the adaptive immune response and at least one gene representative of the immunosuppressive response. The number of genes that may be used in the present method is only limited by the number of distinct biological genes of interest that are practically available at the time of carrying out the method. Accordingly, in one embodiment, a combination of 2, 3, 4, 5, 6, 7, 8, 9, 10, 11, 12, 13, 14, 15, 16, 17, 18, 19, 20, 21, 22, 23, 24, 25, 26, 27, 28, 29distinct genes are quantified, preferably a combination of 2, 3, 4, 5, 6, 7, 8, 9, or 10 genes and more preferably a combination of 2, 3, 4, 5, or 6, genes. However, the number of combined genes that are required for reaching a high statistical relevance (e.g. P value lower than 10⁻³), will be depending on the technique used for quantifying the combination of genes. The number of genes used as genes representative of the adaptive immune response and the number of genes used as genes representative of the immunosuppressive response may be the same or different.

Under preferred conditions of implementation of the invention, an about balanced number of genes of each kind (adaptive immune response and immunosuppressive response) is used, for example 2 of each, or three of each, or 5 of one kind and 6 of the other kind.

Determining an expression level of a gene in a tumor sample obtained from a patient can be implemented by a panel of techniques well known in the art.

Typically, an expression level of a gene is assessed by determining the quantity of mRNA produced by this gene. A subject of the present application is therefore a a method for assessing the responsiveness to anticancer treatment of patients with a cancer defined above comprising determining an expression level ELA of one or several genes representative of human adaptive immune response or the expression level ELI of one or several genes representative of human immunosuppressive response by determining the quantity of mRNA corresponding to said genes.

Methods for determining a quantity of mRNA are well known in the art. For example nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The thus extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA), quantitative new generation sequencing of RNA (NGS).

Nucleic acids (polynucleotides) comprising at least 10 nucleotides and exhibiting sequence complementarity or homology to the mRNA of interest herein find utility as hybridization probes or amplification primers. It is understood that such nucleic acids need not be completely identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500 nucleotides. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

Nucleic acids which may be used as primers or probes in the above amplification and detection method may be assembled as a kit. Such a kit includes consensus primers and molecular probes. A preferred kit also includes the components necessary to determine if amplification has occurred. A kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from cumulus cells and subjecting the RNAs to amplification and hybridization to specific probes, more particularly by means of a quantitative or semi-quantitative RT-PCR.

Probes made using the disclosed methods can be used for nucleic acid detection, such as in situ hybridization (ISH) procedures (for example, fluorescence in situ hybridization (FISH), chromogenic in situ hybridization (CISH) and silver in situ hybridization (SISH)) or comparative genomic hybridization (CGH).

In situ hybridization (ISH) involves contacting a sample containing target nucleic acid sequence (e.g., genomic target nucleic acid sequence) in the context of a metaphase or interphase chromosome preparation (such as a cell or tissue sample mounted on a slide) with a labeled probe specifically hybridizable or specific for the target nucleic acid sequence (e.g., genomic target nucleic acid sequence). The slides are optionally pretreated, e.g., to remove paraffin or other materials that can interfere with uniform hybridization. The sample and the probe are both treated, for example by heating to denature the double stranded nucleic acids. The probe (formulated in a suitable hybridization buffer) and the sample are combined, under conditions and for sufficient time to permit hybridization to occur (typically to reach equilibrium). The chromosome preparation is washed to remove excess probe, and detection of specific labeling of the chromosome target is performed using standard techniques.

For example, a biotinylated probe can be detected using fluorescein-labeled avidin or avidin-alkaline phosphatase. For fluorochrome detection, the fluorochrome can be detected directly, or the samples can be incubated, for example, with fluorescein isothiocyanate (FITC)-conjugated avidin. Amplification of the FITC signal can be effected, if necessary, by incubation with biotin-conjugated goat antiavidin antibodies, washing and a second incubation with FITC-conjugated avidin. For detection by enzyme activity, samples can be incubated, for example, with streptavidin, washed, incubated with biotin-conjugated alkaline phosphatase, washed again and pre-equilibrated (e.g., in alkaline phosphatase (AP) buffer). For a general description of in situ hybridization procedures, see, e.g., U.S. Pat. No. 4,888,278.

Numerous procedures for FISH, CISH, and SISH are known in the art. For example, procedures for performing FISH are described in U.S. Pat. Nos. 5,447,841; 5,472,842; and 5,427,932; and for example, in Pinkel et al., Proc. Natl. Acad. Sci. 83:2934-2938, 1986; Pinkel et al., Proc. Natl. Acad. Sci. 85:9138-9142, 1988; and Lichter et al., Proc. Natl. Acad. Sci. 85:9664-9668, 1988. CISH is described in, e.g., Tanner et al., Am. J. Pathol. 157:1467-1472, 2000 and U.S. Pat. No. 6,942,970. Additional detection methods are provided in U.S. Pat. No. 6,280,929.

Numerous reagents and detection schemes can be employed in conjunction with FISH, CISH, and SISH procedures to improve sensitivity, resolution, or other desirable properties. As discussed above probes labeled with fluorophores (including fluorescent dyes and QUANTUM DOTS®) can be directly optically detected when performing FISH. Alternatively, the probe can be labeled with a nonfluorescent molecule, such as a hapten (such as the following non-limiting examples: biotin, digoxigenin, DNP, and various oxazoles, pyrrazoles, thiazoles, nitroaryls, benzofurazans, triterpenes, ureas, thioureas, rotenones, coumarin, courmarin-based compounds, Podophyllotoxin, Podophyllotoxin-based compounds, and combinations thereof), ligand or other indirectly detectable moiety. Probes labeled with such non-fluorescent molecules (and the target nucleic acid sequences to which they bind) can then be detected by contacting the sample (e.g., the cell or tissue sample to which the probe is bound) with a labeled detection reagent, such as an antibody (or receptor, or other specific binding partner) specific for the chosen hapten or ligand. The detection reagent can be labeled with a fluorophore (e.g., QUANTUM DOT®) or with another indirectly detectable moiety, or can be contacted with one or more additional specific binding agents (e.g., secondary or specific antibodies), which can be labeled with a fluorophore.

In other examples, the probe, or specific binding agent (such as an antibody, e.g., a primary antibody, receptor or other binding agent) is labeled with an enzyme that is capable of converting a fluorogenic or chromogenic composition into a detectable fluorescent, colored or otherwise detectable signal (e.g., as in deposition of detectable metal particles in SISH). As indicated above, the enzyme can be attached directly or indirectly via a linker to the relevant probe or detection reagent. Examples of suitable reagents (e.g., binding reagents) and chemistries (e.g., linker and attachment chemistries) are described in U.S. Patent Application Publications Nos. 2006/0246524; 2006/0246523, and 2007/0117153.

It will be appreciated by those of skill in the art that by appropriately selecting labelled probe-specific binding agent pairs, multiplex detection schemes can be produced to facilitate detection of multiple target nucleic acid sequences (e.g., genomic target nucleic acid sequences) in a single assay (e.g., on a single cell or tissue sample or on more than one cell or tissue sample). For example, a first probe that corresponds to a first target sequence can be labelled with a first hapten, such as biotin, while a second probe that corresponds to a second target sequence can be labelled with a second hapten, such as DNP. Following exposure of the sample to the probes, the bound probes can be detected by contacting the sample with a first specific binding agent (in this case avidin labelled with a first fluorophore, for example, a first spectrally distinct QUANTUM DOT®, e.g., that emits at 585 mn) and a second specific binding agent (in this case an anti-DNP antibody, or antibody fragment, labelled with a second fluorophore (for example, a second spectrally distinct QUANTUM DOT®, e.g., that emits at 705 mn). Additional probes/binding agent pairs can be added to the multiplex detection scheme using other spectrally distinct fluorophores. Numerous variations of direct, and indirect (one step, two step or more) can be envisioned, all of which are suitable in the context of the disclosed probes and assays.

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from cumulus cells and subjecting the RNAs to amplification and hybridization to specific probes, more particularly by means of a quantitative or semi-quantitative RT-PCR.

In another preferred embodiment, the expression level is determined by DNA chip analysis. Such DNA chip or nucleic acid micro array consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210).

The expression level of a gene may be expressed as absolute expression level or normalized expression level. Both types of values may be used in the present method. The expression level of a gene is preferably expressed as normalized expression level when quantitative PCR is used as method of assessment of the expression level because small differences at the beginning of an experiment could provide huge differences after a number of cycles.

Typically, expression levels are normalized by correcting the absolute expression level of a gene by comparing its expression to the expression of a gene that is not relevant for determining the cancer stage of the patient, e.g., a housekeeping gene that is constitutively expressed. Suitable genes for normalization include housekeeping genes such as the actin gene ACTB, ribosomal 18S gene, GUSB, PGK1 and TFRC. This normalization allows comparing the expression level of one sample, e.g., a patient sample, with the expression level of another sample, or comparing samples from different sources.

The present method includes comparing the expression levels ELA₁ - ELAₙ and ELI₁ - ELIₙ determined at step i) with predetermined reference values ELRA₁ - ELRAₙ and ELRI₁ - ELRIₙ (collectively named ELR). The predetermined reference values ELRA₁ - ELRAₙ and ELRI₁ - ELRIₙ may be a definite value or a range of values.

For example, if the expression level ELA₁ of a tumour sample of a patient for the genes considered is higher than the corresponding predetermined reference value (or range of values) ELRA₁, the patient will be considered as "good responder" ("High" or any such assessment such as "positive", etc.) and will be considered as "bad responder" ("Low" or any such assessment such as "negative", etc.), if ELA₁ is lower than ELRA₁. Similar considerations apply to ELI₁ - ELIₙ and ELRI₁ - ELRIₙ.

Predetermined reference values used for comparison may consist of "cut-off' values that may be determined as described in WO2007045996.

Predetermined reference values used for comparison may consist of "cut-off' values that may be determined as described hereunder. Each reference ("cut-off") value ELR for each gene may be determined by carrying out a method comprising the steps of:
a) providing a collection of tumor tissue samples from patients suffering of cancer;
b) determining the expression level of the relevant gene for each tumour tissue sample contained in the collection provided at step a);
c) ranking the tumor tissue samples according to said expression level
d) classifying said tumour tissue samples in pairs of subsets of increasing, respectively decreasing, number of members ranked according to their expression level,
e) providing, for each tumour tissue sample provided at step a), information relating to the actual clinical outcome for the corresponding cancer patient (i.e. the duration of the disease-free survival (DFS) or the overall survival (OS) or both);
f) for each pair of subsets of tumour tissue samples, obtaining a Kaplan Meier percentage of survival curve;
g) for each pair of subsets of tumour tissue samples calculating the statistical significance (p value) between both subsets
h) selecting as reference value ELR for the expression level, the value of expression level for which the p value is the smallest.

A confidence interval may be constructed around the value of expression level thus obtained, for example ELR ± 5 or 10%.

For example the expression level of a gene G1 has been assessed for 100 cancer samples of 100 patients. The 100 samples are ranked according to the expression level of gene G1. Sample 1 has the highest expression level and sample 100 has the lowest expression level. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding cancer patient, Kaplan Meier curves are prepared for each of the 99 groups of two subsets. Also for each of the 99 groups, the p value between both subsets was calculated.

The reference value ELR is selected such as the discrimination based on the criterion of the minimum p value is the strongest. In other terms, the expression level corresponding to the boundary between both subsets for which the p value is minimum is considered as the reference value. It should be noted that according to the experiments made by the inventors, the reference value ELR is not necessarily the median value of expression levels.

In routine work, the reference value ELR (cut-off value) may be used in the present method to discriminate tumour samples and therefore the corresponding patients.

Kaplan-Meier curves of percentage of survival as a function of time are commonly used to measure the fraction of patients living for a certain amount of time after treatment and are well known by the man skilled in the art. P value is conventionally used in statistical significance testing.

The man skilled in the art also understands that the same technique of assessment of the expression level of a gene should preferably be used for obtaining the reference value and thereafter for assessment of the expression level of a gene of a patient subjected to the method of the invention.

Such predetermined reference values of expression level may be determined for any gene defined above and may be used in the method of the invention for screening patients with a cancer. A reference value ELRA for one or for each of several genes representative of human adaptive immune response and a reference value ELRI for one or each of several genes representative of human immunosuppressive response are necessary for implementing the method of the invention.

A tumor tissue sample from a patient suffering of cancer may be subjected to determination of the expression levels ELA₁ - ELAₙ and ELI₁ - ELIₙ respectively of genes GA₁ - GAₙ and GI₁ - GIₙ 1 by using the same technique as the technique used for obtaining reference values ELRA₁ - ELRAₙ and ELRI₁ - ELRIₙ, for example by determining the amount of mRNA (for exemple using Quantitative PCR) produced by the relevant gene. Different techniques may be used for obtaining the relevant data for two different genes or several different genes. However, preferably a same technique is implemented, preferably selected among those previously cited or used in the examples hereafter.

If for example ELA1 is higher than ELRAI, the patient is considered as a good responder as regards human adaptive immune response and as a bad responder if ELA1 is lower than ELRA1. Similarly, if ELI1 is higher than ELRI1, the patient is considered as a good responder as regards human immunosuppressive response and as a bad responder if ELI1 is lower than ELRI1.

Of particular note is the fact that according to the technique of assessment of the expression level, a numerical value lower than the reference value may actually mean that the expression level is higher than the reference level. For example, in the examples thereafter, using real-time PCR, a dCt value lower than the relevant reference value means that the signal was detected earlier, i.e.: the expression level of the gene is higher than the reference level.

The method for screening patients which is the subject matter of the present invention has very advantageous properties and qualities.

The method allows in particular, from the knowledge of the response of the patient concerning human adaptive immune response and human immunosuppressive response, to make a good assessment of prognosis with respect to DFS and OS of a patient, independently of the cancer type, origin or stage as evidenced in the experimental section.

The setting of a single "cut-off' value allows discrimination between a poor and a good prognosis with respect to DFS and OS for a patient. Practically, high statistical significance values (e.g. low P values) are generally obtained for a range of successive arbitrary quantification values, and not only for a single arbitrary quantification value. Thus, in one alternative embodiment of the invention, instead of using a definite reference value ELR, a range of values is provided.

Therefore, a minimal statistical significance value (minimal threshold of significance, e.g. maximal threshold P value) is arbitrarily set and a range of a plurality of arbitrary quantification values for which the statistical significance value calculated at step g) is higher (more significant, e.g. lower P value) are retained, so that a range of quantification values is provided. This range of quantification values includes a "cut-off" value as described above. According to this specific embodiment of a "cut-off' value, poor or good clinical outcome prognosis can be determined by comparing the expression level with the range of values which are identified. In certain embodiments, a cut-off value thus consists of a range of quantification values, e.g. centered on the quantification value for which the highest statistical significance value is found (e.g. generally the minimum P value which is found). For example, on a hypothetical scale of 1 to 10, if the ideal cut-off value (the value with the highest statistical significance) is 5, a suitable (exemplary) range may be from 4-6.

Therefore, a patient may be assessed by comparing values obtained by measuring the expression level of a gene representative of the immune adaptive response and a gene representative of the immunosuppressive response, where values greater than 5 reveal a good prognosis (a contrario a bad prognosis when the gene is representative of immunosuppressive response) and values less than 5 reveal a poor prognosis; (a contrario a good prognosis when the gene is representative of immunosuppressive response). In a another embodiment, a patient may be assessed by comparing values obtained by measuring the expression level of a gene representative of the immune adaptive response and a gene representative of the immunosuppressive response and comparing the values on a scale, where values above the range of 4-6 indicate a good prognosis (a contrario a bad prognosis when the gene is representative of immunosuppressive response) and values below the range of 4-6 indicate a poor prognosis (a contrario a good prognosis when the gene is representative of immunosuppressive response), with values falling within the range of 4-6 indicating an intermediate prognosis.

The method according to the invention comprises the step of concluding that a patient would advantageously receive an antitumoral treatment if the patient is a good responder for each of human adaptive immune response and human immunosuppressive response. According to still another embodiment of the invention, the method comprises the step of concluding that a patient would not advantageously receive an antitumoral treatment if the patient is not a good responder for both of human adaptive immune response and human immunosuppressive response.

In a particular embodiment, the method of the invention comprises comparison steps which include a classification of the quantification values measured for the expression level of a gene into two possibilities, respectively: (i) a first possibility when the quantification value for the expression level is higher than the predetermined corresponding reference value (the first possibility is named "Hi" for example) and (ii) a second possibility when the quantification value for the expression level is lower than the predetermined corresponding reference value (the second possibility is named " Lo " for example).

It flows from the example that if the result of the comparison step consists of a "Hi" value for the gene representative of the immune adaptive response and a "Lo" value for the gene representative of the immunosuppressive response, then a good prognosis is provided and the treatment will provide a limited (or moderate) impact on the survival of the patient (i.e. a "bad responder to antitumoral treatment"). In the same way, if the result of the comparison step consists of a "Lo" value for the gene representative of the immune adaptive response and a "Hi" value for the gene representative of the immunosuppressive response then a poor prognosis is provided and the treatment will provide a limited (or moderate) impact on the survival of the patient (i.e. a "bad responder to antitumoral treatment "). Conversly, if the result of the comparison step consists of a "Hi" value for the gene representative of the immune adaptive response and a "Hi" value for the gene representative of the immunosuppressive response then a intermediate prognosis is provided and the treatment will provide a significant imapact on the survival of the patient (i.e. a "good responder to antitumoral treatment "). For patients without distant metastasis (illustrated for stage II/III patients), the different scenarios are summarized in Table 3 hereafter.

**Table 3: Results for a combination of two genes (a single gene representative of the immune adaptive response and a single gene representative of the immunosuppressive response).**

| Result | Prognosis | Response to treatment | Score |
|---|---|---|---|
| "Hi" for the gene representative of the immune adaptive response and | Good prognosis | Bad responder to antitumoral treatment | 2 |
| "Lo" value for the gene representative of the immunosuppressive response | | | |
| "Hi" for the gene representative of the immune adaptive response and | Intermediate prognosis | Good responder to antitumoral treatment | 0 |
| "Hi" value for the gene representative of the immunosuppressive response | | | |
| "Lo" for the gene representative of the immune adaptive response and | Intermediate bad prognosis | Bad responder to antitumoral treatment | 0 |
| "Lo" value for the gene representative of the immunosuppressive response | | | |
| "Lo" for the gene representative of the immune adaptive response and | Bad prognosis | Bad responder to antitumoral treatment | -2 |
| "Hi" value for the gene representative of the immunosuppressive response | | | |

A score which is a composite of the classifications may also be calculated. For example a positive coefficient (e.g. +1) is allocated when expression level of the gene representative of the immune adaptive response is higher than the predetermined reference value and a negative coefficient (e.g. -1) is allocated when expression level of the gene representative of the immune adaptive response is lower than the predetermined reference value. Conversely, a positive coefficient (e.g. +1) is allocated when expression level of the gene representative of the immunosupressive response is lower than the predetermined reference value and a negative coefficient (e.g. -1) is allocated when expression level of the gene representative of the immunosupressive response is higher than the predetermined reference value.

Calculating a score is particularly suitable to make easier to understand the results of the comparison step when a combination of more than 2 genes is used. Typically the more such a score is close to the value 0, the more the treatment will have a positive effect on the survival of the patient (i.e. the patient would benefit of the treatment).

The present method therefore allows defining inter alia a new group of patients which had never been identified until now, i.e. patients whose cancer will be successfully treated by an anti-cancer treatment.

An anti-cancer treatment may consist of radiotherapy, chemotherapy or immunotherapy. The treatment may consist of an adjuvant therapy (i.e. treatment after chirurgical resection of the primary tumor) of a neoadjuvant therapy (i.e. treatment before chirurgical resection of the primary tumor).

The present invention therefore relates to a chemotherapeutic agent, a radiotherapeutic agent, or an immunotherapeutic agent, preferably the latter, for use in the treatment of a stage I-III cancer patient who has been considered as a good responder to antitumoral treatment according to the above method of the invention.

The term "chemotherapeutic agent" refers to chemical compounds that are effective in inhibiting tumor growth. Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estrarnustine, ifosfamide, mechlorethamine, mechlorethamine oxide hydrochloride, melphalan, novembichin, phenesterine, prednimus tine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin (11 and calicheamicin 211, see, e.g., Agnew Chem Intl. Ed. Engl. 33:183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, canninomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idanrbicin, marcellomycin, mitomycins, mycophenolic acid, nogalarnycin, olivomycins, peplomycin, potfiromycin, puromycin, quelamycin, rodorubicin, streptomgrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophospharnide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pento statin; phenamet; pirarubicin; podophyllinic acid; 2-ethylhydrazide; procarbazine; PSK®; razoxane; rhizoxin; sizofiran; spirogennanium; tenuazonic acid; triaziquone; 2,2',2"-trichlorotriethylarnine; trichothecenes (especially T-2 toxin, verracurin A, roridinA and anguidine); urethan; vindesine; dacarbazine; mannomustine; mitobromtol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, N.].) and doxetaxel (TAXOTERE®, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-1 1 ; topoisomerase inhibitor RFS 2000; difluoromethylornithine (DMFO); retinoic acid; capecitabine; and phannaceutically acceptable salts, acids or derivatives of any of the above. Also included in this definition are antihormonal agents that act to regulate or inhibit honnone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and phannaceutically acceptable salts, acids or derivatives of any of the above.

The term "immunotherapeutic agent," as used herein, refers to a compound, composition or treatment that indirectly or directly enhances, stimulates or increases the body's immune response against cancer cells and/or that decreases the side effects of other anticancer therapies. Immunotherapy is thus a therapy that directly or indirectly stimulates or enhances the immune system's responses to cancer cells and/or lessens the side effects that may have been caused by other anti-cancer agents. Immunotherapy is also referred to in the art as immunologic therapy, biological therapy biological response modifier therapy and biotherapy. Examples of common immunotherapeutic agents known in the art include, but are not limited to, cytokines, cancer vaccines, monoclonal antibodies and non-cytokine adjuvants. Alternatively the immunotherapeutic treatment may consist of administering the patient with an amount of immune cells (T cells, NK, cells, dendritic cells, B cells...).

Immunotherapeutic agents can be non-specific, i.e. boost the immune system generally so that the human body becomes more effective in fighting the growth and/or spread of cancer cells, or they can be specific, i.e. targeted to the cancer cells themselves immunotherapy regimens may combine the use of non-specific and specific immunotherapeutic agents.

Non-specific immunotherapeutic agents are substances that stimulate or indirectly improve the immune system. Non-specific immunotherapeutic agents have been used alone as a main therapy for the treatment of cancer, as well as in addition to a main therapy, in which case the non-specific immunotherapeutic agent functions as an adjuvant to enhance the effectiveness of other therapies (e.g. cancer vaccines). Non-specific immunotherapeutic agents can also function in this latter context to reduce the side effects of other therapies, for example, bone marrow suppression induced by certain chemotherapeutic agents. Non-specific immunotherapeutic agents can act on key immune system cells and cause secondary responses, such as increased production of cytokines and immunoglobulins. Alternatively, the agents can themselves comprise cytokines. Non-specific immunotherapeutic agents are generally classified as cytokines or non-cytokine adjuvants.

A number of cytokines have found application in the treatment of cancer either as general non-specific immunotherapies designed to boost the immune system, or as adjuvants provided with other therapies. Suitable cytokines include, but are not limited to, interferons, interleukins and colony-stimulating factors.

Interferons (IFNs) contemplated by the present invention include the common types of IFNs, IFN-alpha (IFN-a), IFN-beta (IFN-beta) and IFN-gamma (IFN-y). IFNs can act directly on cancer cells, for example, by slowing their growth, promoting their development into cells with more normal behaviour and/or increasing their production of antigens thus making the cancer cells easier for the immune system to recognise and destroy. IFNs can also act indirectly on cancer cells, for example, by slowing down angiogenesis, boosting the immune system and/or stimulating natural killer (NK) cells, T cells and macrophages. Recombinant IFN-alpha is available commercially as Roferon (Roche Pharmaceuticals) and Intron A (Schering Corporation). The use of IFN-alpha, alone or in combination with other immunotherapeutics or with chemotherapeutics, has shown efficacy in the treatment of various cancers including melanoma (including metastatic melanoma), renal cancer (including metastatic renal cancer), breast cancer, prostate cancer, and cervical cancer (including metastatic cervical cancer).

Interleukins contemplated by the present invention include IL-2, IL-4, IL-11 and IL-12. Examples of commercially available recombinant interleukins include Proleukin® (IL-2; Chiron Corporation) and Neumega® (IL-12; Wyeth Pharmaceuticals). Zymogenetics, Inc. (Seattle, Wash.) is currently testing a recombinant form of IL-21, which is also contemplated for use in the combinations of the present invention. Interleukins, alone or in combination with other immunotherapeutics or with chemotherapeutics, have shown efficacy in the treatment of various cancers including renal cancer (including metastatic renal cancer), melanoma (including metastatic melanoma), ovarian cancer (including recurrent ovarian cancer), cervical cancer (including metastatic cervical cancer), breast cancer, colorectal cancer, lung cancer, brain cancer, and prostate cancer.

Interleukins have also shown good activity in combination with IFN-alpha in the treatment of various cancers (Negrier et al., Ann Oncol. 2002 13(9):1460-8 ; Touranietal, J. Clin. Oncol. 2003 21(21):398794).

Colony-stimulating factors (CSFs) contemplated by the present invention include granulocyte colony stimulating factor (G-CSF or filgrastim), granulocyte-macrophage colony stimulating factor (GM-CSF or sargramostim) and erythropoietin (epoetin alfa, darbepoietin). Treatment with one or more growth factors can help to stimulate the generation of new blood cells in patients undergoing traditional chemotherapy. Accordingly, treatment with CSFs can be helpful in decreasing the side effects associated with chemotherapy and can allow for higher doses of chemotherapeutic agents to be used. Various-recombinant colony stimulating factors are available commercially, for example, Neupogen® (G-CSF; Amgen), Neulasta (pelfilgrastim; Amgen), Leukine (GM-CSF; Berlex), Procrit (erythropoietin; Ortho Biotech), Epogen (erythropoietin; Amgen), Arnesp (erytropoietin). Colony stimulating factors have shown efficacy in the treatment of cancer, including melanoma, colorectal cancer (including metastatic colorectal cancer), and lung cancer.

Non-cytokine adjuvants suitable for use in the combinations of the present invention include, but are not limited to, Levamisole, alum hydroxide (alum), Calmette-Guerin bacillus (ACG), incomplete Freund's Adjuvant (IFA), QS-21, DETOX, Keyhole limpet hemocyanin (KLH) and dinitrophenyl (DNP). Non-cytokine adjuvants in combination with other immuno- and/or chemotherapeutics have demonstrated efficacy against various cancers including, for example, colon cancer and colorectal cancer (Levimasole); melanoma (BCG and QS-21); renal cancer and bladder cancer (BCG).

In addition to having specific or non-specific targets, immunotherapeutic agents can be active, i.e. stimulate the body's own immune response, or they can be passive, i.e. comprise immune system components that were generated external to the body.

Passive specific immunotherapy typically involves the use of one or more monoclonal antibodies that are specific for a particular antigen found on the surface of a cancer cell or that are specific for a particular cell growth factor. Monoclonal antibodies may be used in the treatment of cancer in a number of ways, for example, to enhance a subject's immune response to a specific type of cancer, to interfere with the growth of cancer cells by targeting specific cell growth factors, such as those involved in angiogenesis, or by enhancing the delivery of other anticancer agents to cancer cells when linked or conjugated to agents such as chemotherapeutic agents, radioactive particles or toxins.

Monoclonal antibodies currently used as cancer immunotherapeutic agents that are suitable for inclusion in the combinations of the present invention include, but are not limited to, rituximab (Rituxan®), trastuzumab (Herceptin®), ibritumomab tiuxetan (Zevalin®), tositumomab (Bexxar®), cetuximab (C-225, Erbitux®), bevacizumab (Avastin®), gemtuzumab ozogamicin (Mylotarg®), alemtuzumab (Campath®), and BL22. Monoclonal antibodies are used in the treatment of a wide range of cancers including breast cancer (including advanced metastatic breast cancer), colorectal cancer (including advanced and/or metastatic colorectal cancer), ovarian cancer, lung cancer, prostate cancer, cervical cancer, melanoma and brain tumours. Other examples include anti-CTLA4 antibodies (e.g. Ipilimumab), anti-PDl antibodies, anti-PDLl antibodies, anti-TIMP3 antibodies, anti-LAG3 antibodies, anti-B7H3 antibodies, anti-B7H4 antibodies or anti-B7H6 antibodies.

Monoclonal antibodies can be used alone or in combination with other immunotherapeutic agents or chemotherapeutic agents.

Active specific immunotherapy typically involves the use of cancer vaccines. Cancer vaccines have been developed that comprise whole cancer cells, parts of cancer cells or one or more antigens derived from cancer cells. Cancer vaccines, alone or in combination with one or more immuno- or chemotherapeutic agents are being investigated in the treatment of several types of cancer including melanoma, renal cancer, ovarian cancer, breast cancer, colorectal cancer, and lung cancer. Non-specific immunotherapeutics are useful in combination with cancer vaccines in order to enhance the body's immune response.

The immunotherapeutic treatment may consist of an adoptive immunotherapy as described by Nicholas P. Restifo, Mark E. Dudley and Steven A. Rosenberg "Adoptive immunotherapy for cancer: harnessing the T cell response, Nature Reviews Immunology, Volume 12, April 2012). In adoptive immunotherapy, the patient's circulating lymphocytes, or tumor infiltrated lymphocytes, are isolated in vitro, activated by lymphokines such as IL-2 or transuded with genes for tumor necrosis, and readministered (Rosenberg et al., 1988; 1989). The activated lymphocytes are most preferably be the patient's own cells that were earlier isolated from a blood or tumor sample and activated (or "expanded") in vitro. This form of immunotherapy has produced several cases of regression of melanoma and renal carcinoma.

The term "radiotherapeutic agent" as used herein, is intended to refer to any radiotherapeutic agent known to one of skill in the art to be effective to treat or ameliorate cancer, without limitation. For instance, the radiotherapeutic agent can be an agent such as those administered in brachytherapy or radionuclide therapy. Such methods can optionally further comprise the administration of one or more additional cancer therapies, such as, but not limited to, chemotherapies, and/or another radiotherapy.

A further object of the invention relates to the use of kits for performing the methods of the invention, wherein said kits comprise means for measuring the expression level of the gene clusters of the invention in the sample obtained from the patient.

A kit may include probes, primers macroarrays or microarrays as above described. For example, the kit may comprise a set of probes as above defined, usually made of DNA, and that may be pre-labelled. Alternatively, probes may be unlabelled and the ingredients for labelling may be included in the kit in separate containers. A kit may further comprise hybridization reagents or other suitably packaged reagents and materials needed for the particular hybridization protocol, including solid-phase matrices, if applicable, and standards. Alternatively the kit of the invention may comprise amplification primers that may be pre-labelled or may contain an affinity purification or attachment moiety. The kit may further comprise amplification reagents and also other suitably packaged reagents and materials needed for the particular amplification protocol.

As an exemple, based on the statistical data of Table 5, a kit composed of the 3 adaptive genes, CD3G + CCR2 + GZMA and of the 3 suppressive genes REN + PDCD1 + VEGF, will be particularly suitable for predicting response to therapy, including anti-VEGF treatment (Avastin) in particular with the combination GZMA/VEGF, anti-PDl treatment in particular with the combinations CD3G/PDCD1, CCR2/PDCD1, GZMA/PDCD1, or adjuvant chemotherapy as illustrated by the P-values in Table 5.

The invention will be further illustrated by the following figures and examples. These examples and figures should not be interpreted in any way as limiting the scope of the present invention.

### FIGURES:

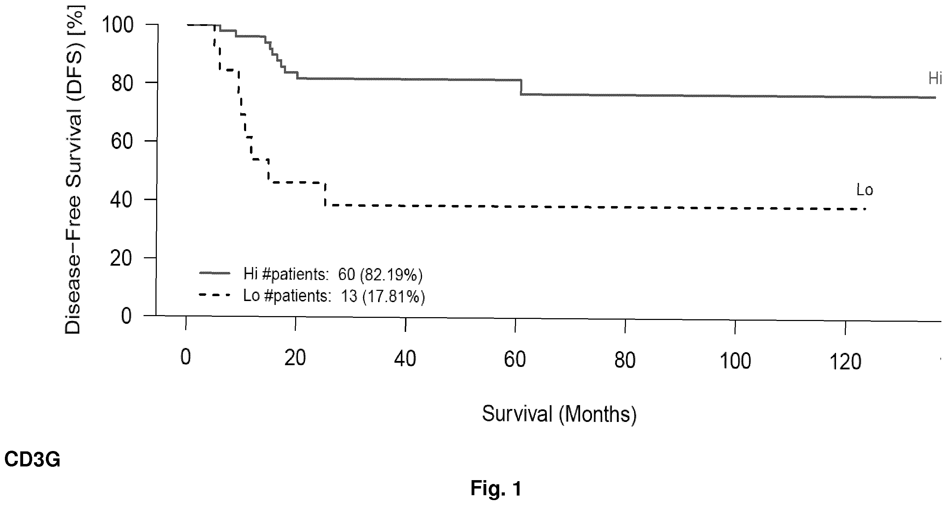

Figures 1, 2 and 3 represent Kaplan Meier curves (percentage of disease free survival versus time in months) concerning 73 pairs of subsets such as mentioned in preparation 1, for genes CD3G, GZMH and ITGAE and for their corresponding optimal reference value.

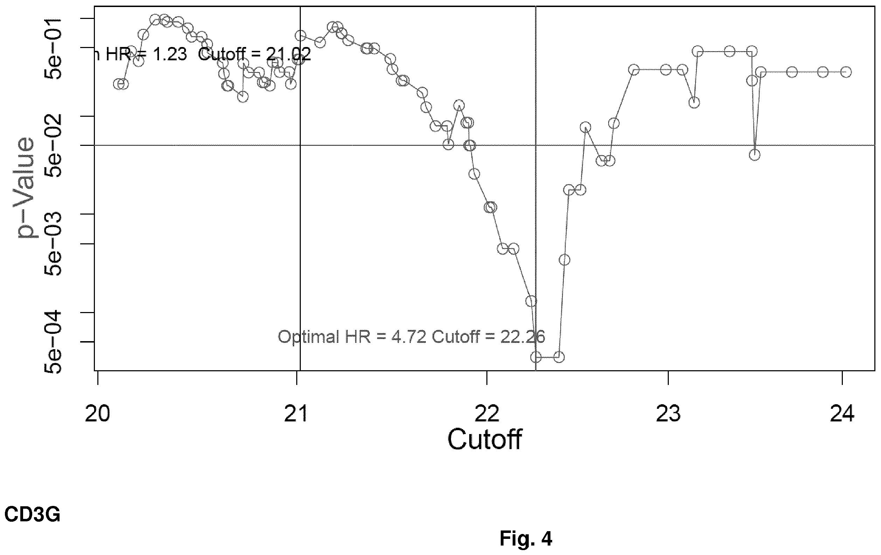

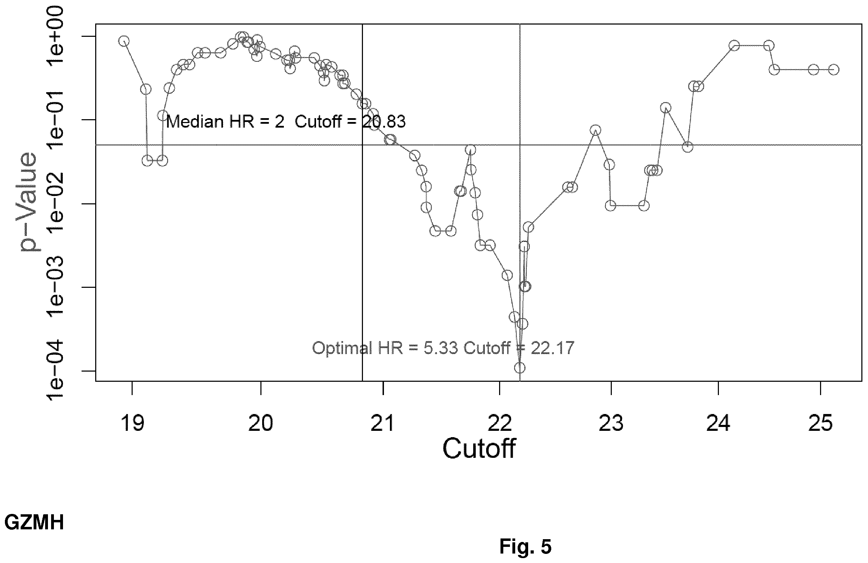

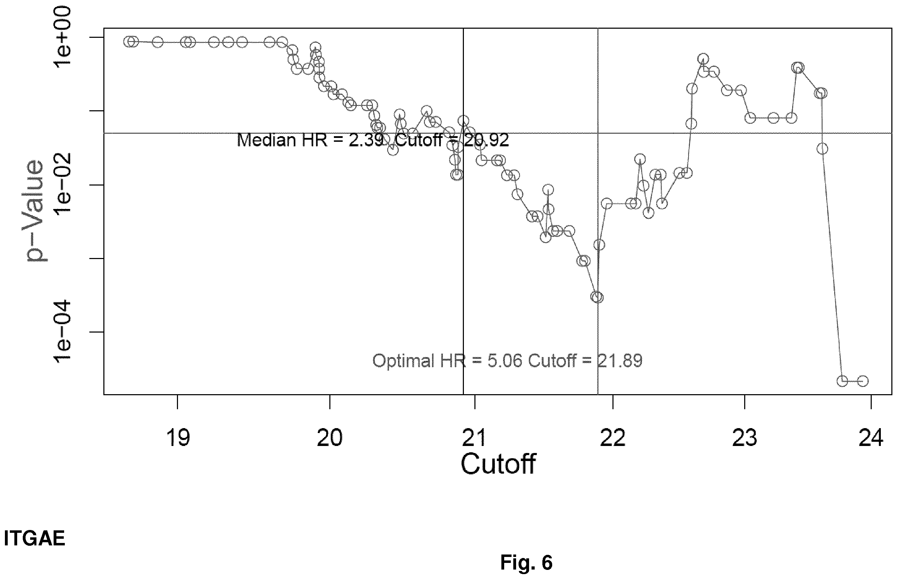

Figures 4, 5 and 6 represent curves of p values (ordinate axis scale 1e-04 to 1e-00), versus expression level values (horizontal axis as abscissa). Vertical lines crossing the x-axis (at 22.26 and at 21.05 on Figure 4) represent the optimal and median cut-points respectively. Optimal HR represents the value of the Hazard Ratio (on Disease-free survival curves) at the optimal cut-point.

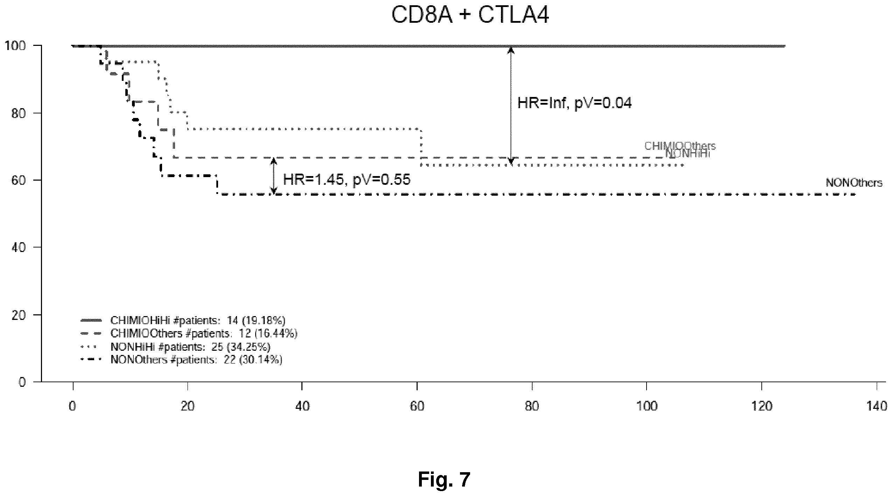

Figure 7 represents a Kaplan Meier curve for disease free survival (DFS) of Stage II/III colorectal cancer patients. Four groups are represented, two subsets of patients were submitted to chemotherapies (CHIMIO) and the two other subsets were not treated (NON). Patients HiHi have a high expression of both genes (CD8A and CTLA4) compared to all other patients ("Others").

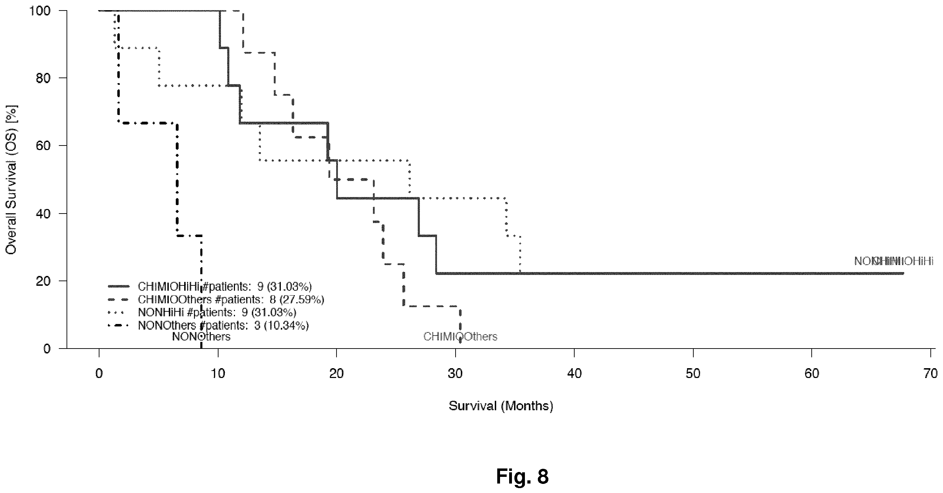

Figure 8 represents a Kaplan Meier curve for four subsets of colorectal cancer Stage IV patients. Two subsets of patients were submitted to chemotherapies (CHIMIO) and the two other subsets were not treated (NON). Patients HiHi have a high expression of both genes compared to all other patients ("Others").
Figure 9 illustrates combinations of genes of both types. The lines represent correlations between two genes showing similarities of gene expression pattern between patients.

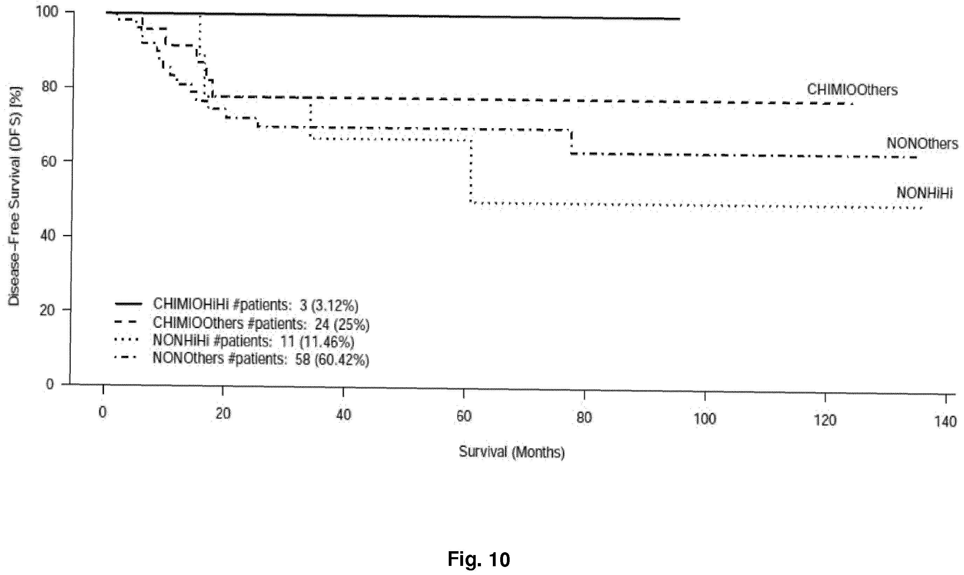

Figure 10 represents a Kaplan Meier curve for disease free survival (DFS) of Stage I, II, III colorectal cancer patients. Four groups are represented, two subsets of patients were submitted to chemotherapies (CHIMIO) and the two other subsets were not treated (NON). Patients HiHi have a high expression of both genes (CD8A and VEGFA) compared to all other patients ("Others").

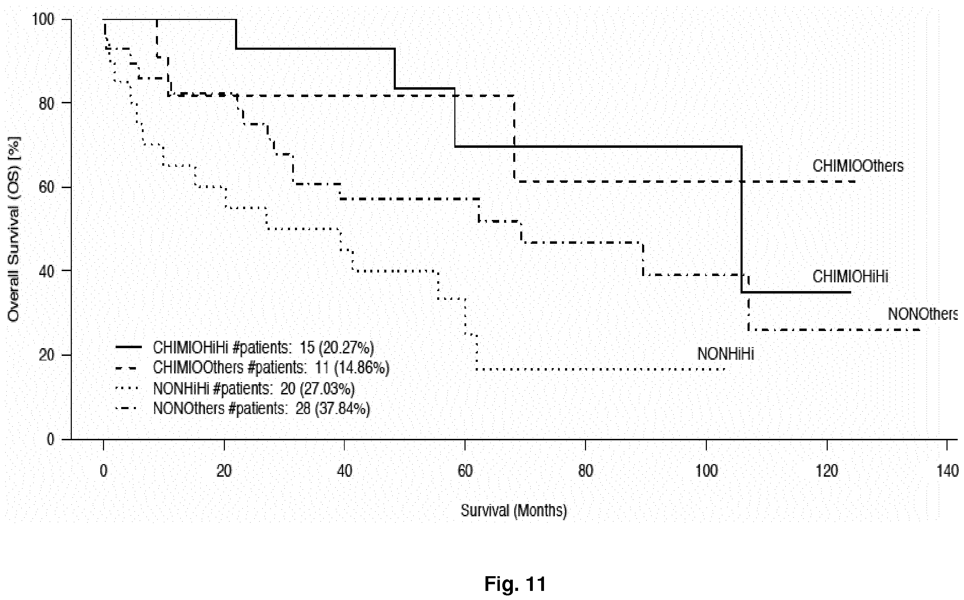

Figure 11 represents a Kaplan Meier curve for overall survival (OS) of Stage II/III colorectal cancer patients. Four groups are represented, two subsets of patients were submitted to chemotherapies (CHIMIO) and the two other subsets were not treated (NON). Patients HiHi have a high expression of both genes (CD3E and VEGFA) compared to all other patients ("Others").

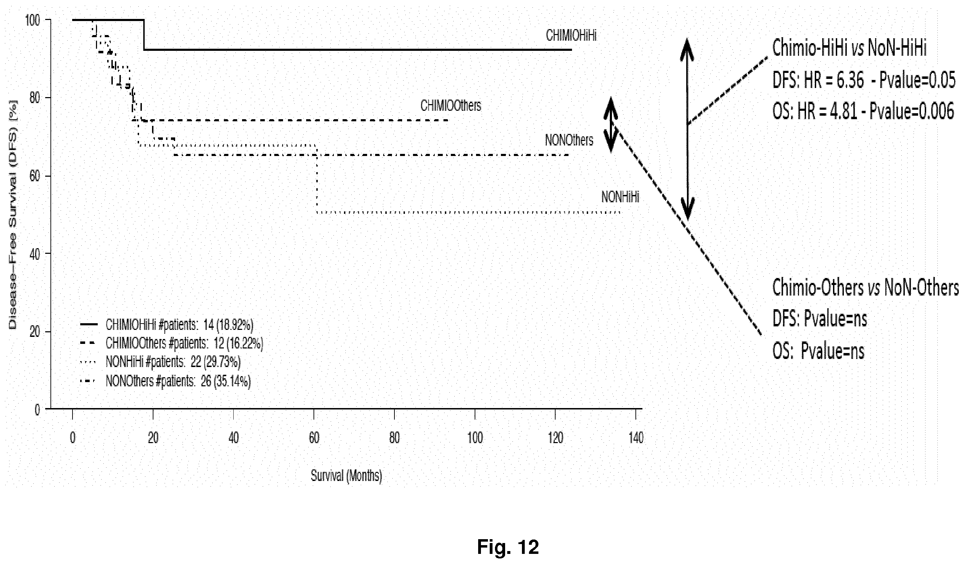

Figure 12 represents a Kaplan Meier curve for disease free survival (DFS) of Stage II/III colorectal cancer patients. Four groups are represented, two subsets of patients were submitted to chemotherapies (CHIMIO) and the two other subsets were not treated (NON). Patients HiHi have a high expression of both genes (CD3G and VEGFA) compared to all other patients ("Others").

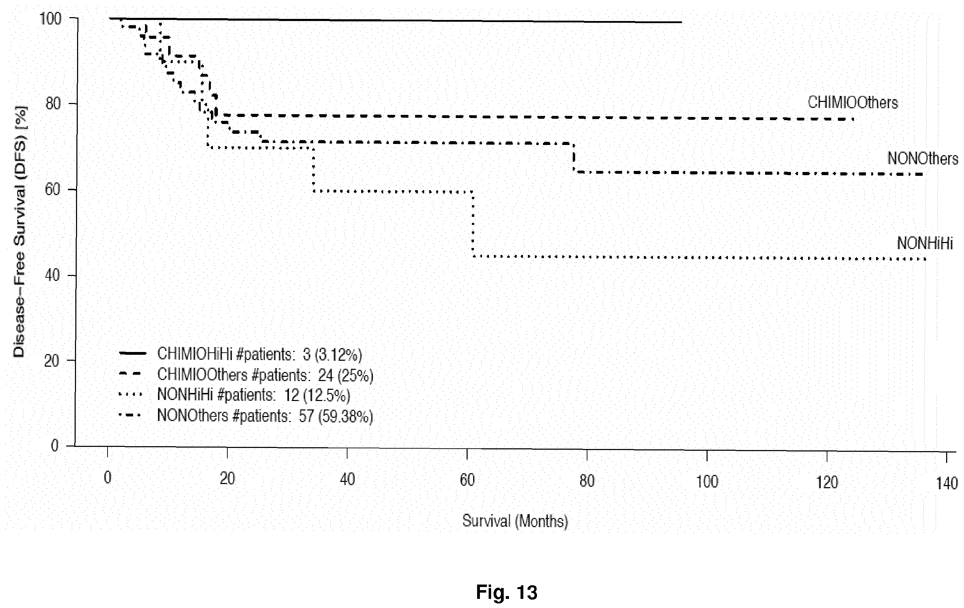

Figure 13 represents a Kaplan Meier curve for disease free survival (DFS) of Stage I, II, III colorectal cancer patients. Four groups are represented, two subsets of patients were submitted to chemotherapies (CHIMIO) and the two other subsets were not treated (NON). Patients HiHi have a high expression of both genes (CD3G and VEGFA) compared to all other patients ("Others").

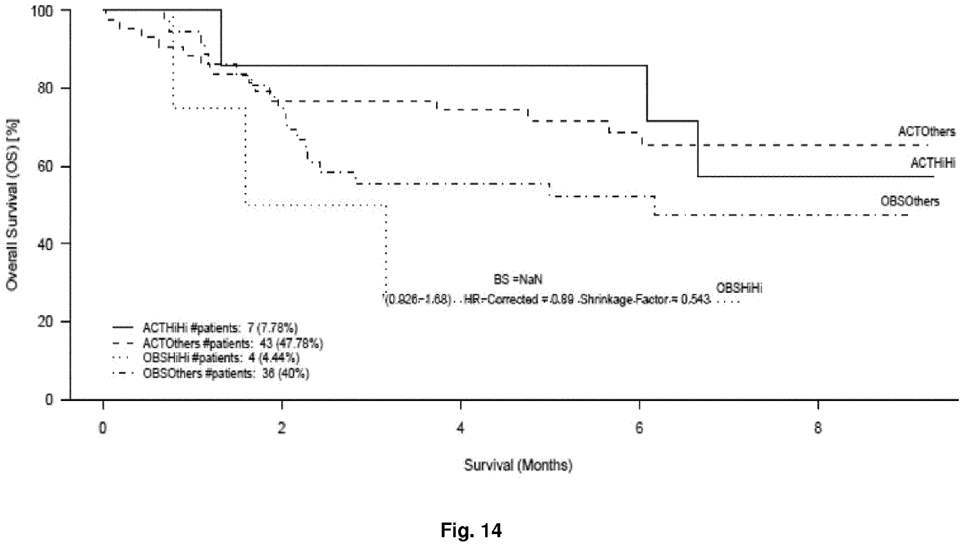

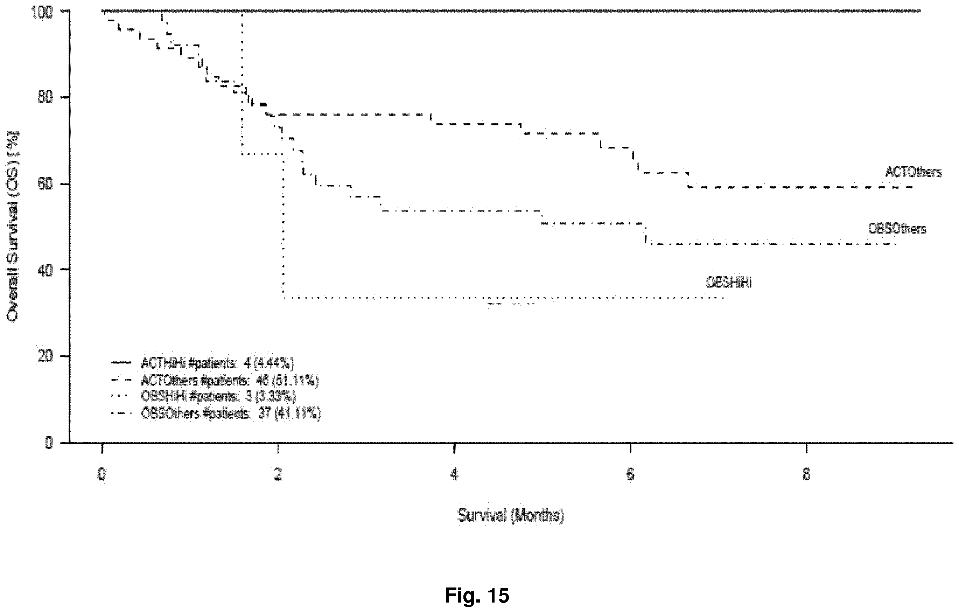

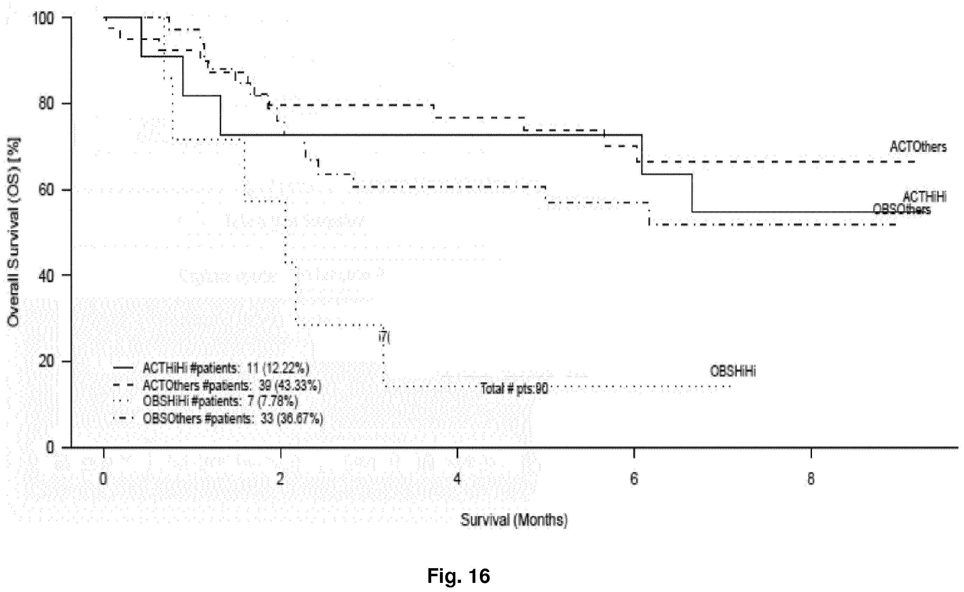

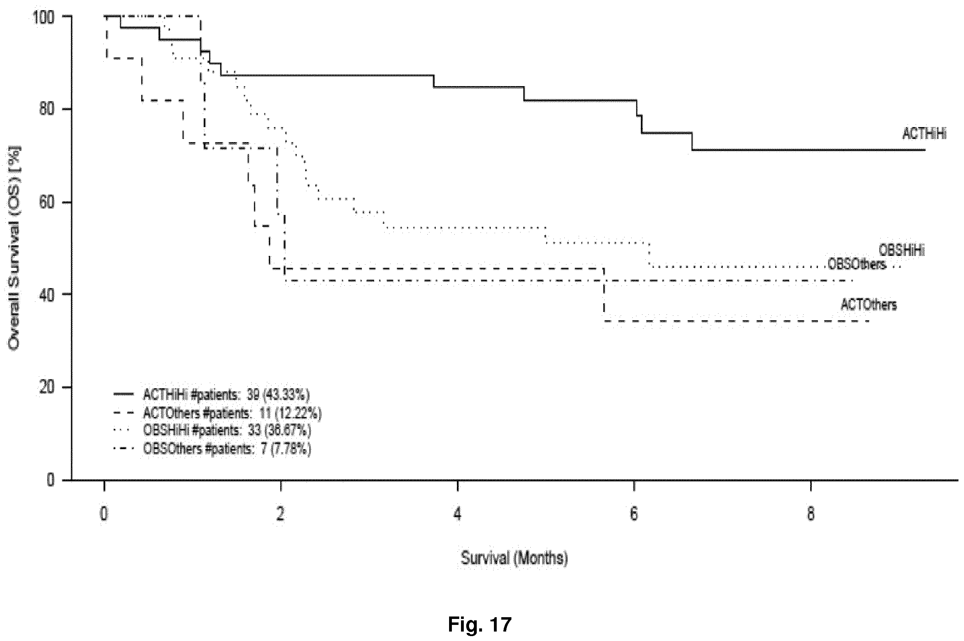

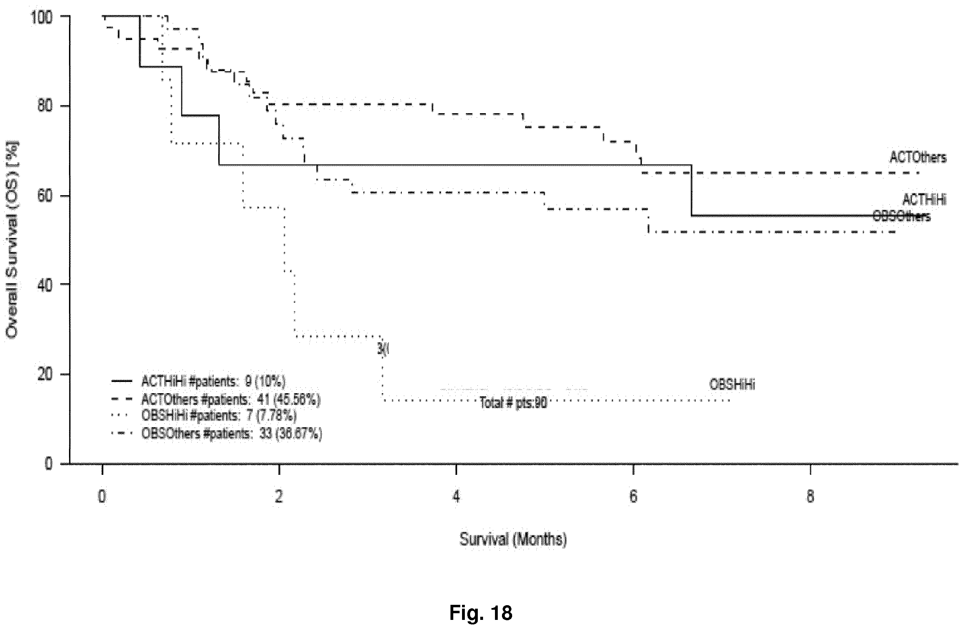

Figures 14-18 represent Kaplan Meier curves for overall survival (OS) of early stage (I, II) lung cancer patients. Four groups are represented, two subsets of patients were submitted to chemotherapies (ACT) and the two other subsets were not treated (OBS). Patients HiHi have a high expression of both genes (Figure14: PDCD1 and CCL2, Figure 15: PDCD1 and CD247, Figure 16: PDCD1 and CD3E, Figure 17: VEGFA and CD3E, Figure18: PDCD1 and CXCL10) compared to all other patients ("Others").

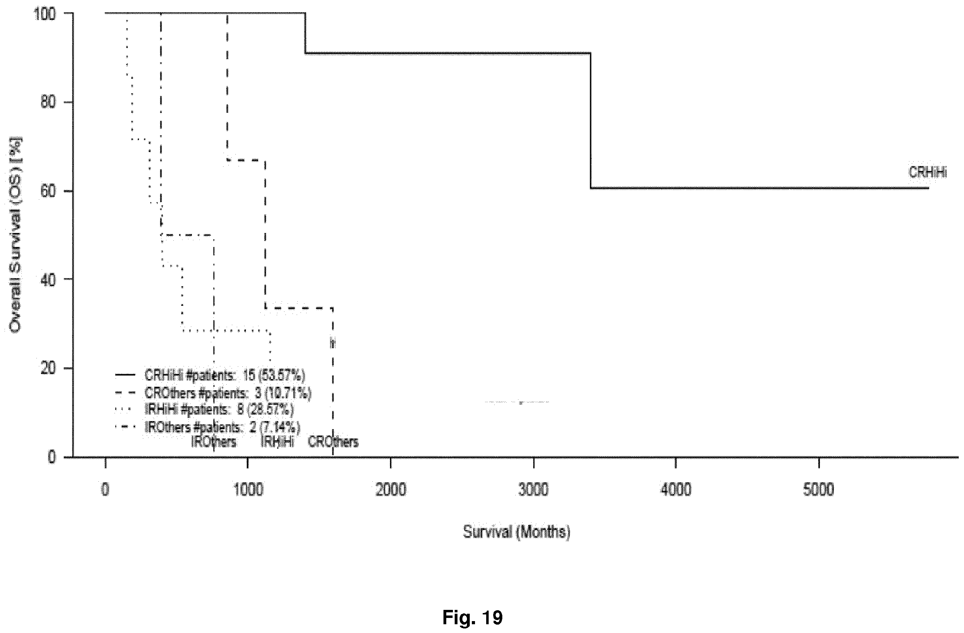

Figure 19 represents a Kaplan Meier curve for disease free survival (DFS) of advanced stage ovarian cancer patients. All patients were submitted to chemotherapy. Four groups are represented, two subsets of patients with complete response (CR) and the two other subsets with no complete response (IR). Patients HiHi have a high expression of both genes (CD1A and CCL2) compared to all other patients ("Others").

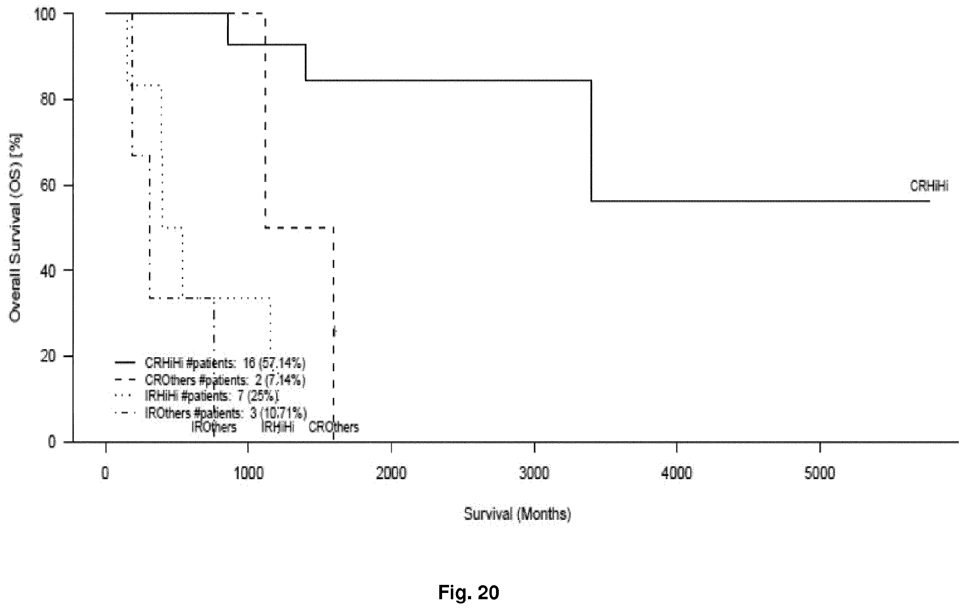

Figure 20 represents a Kaplan Meier curve for disease free survival (DFS) of advanced stage ovarian cancer patients. All patients were submitted to chemotherapy. Four groups are represented, two subsets of patients with complete response (CR) and the two other subsets with no complete response (IR). Patients HiHi have a high expression of both genes (CD1A and CX3CL1) compared to all other patients ("Others").

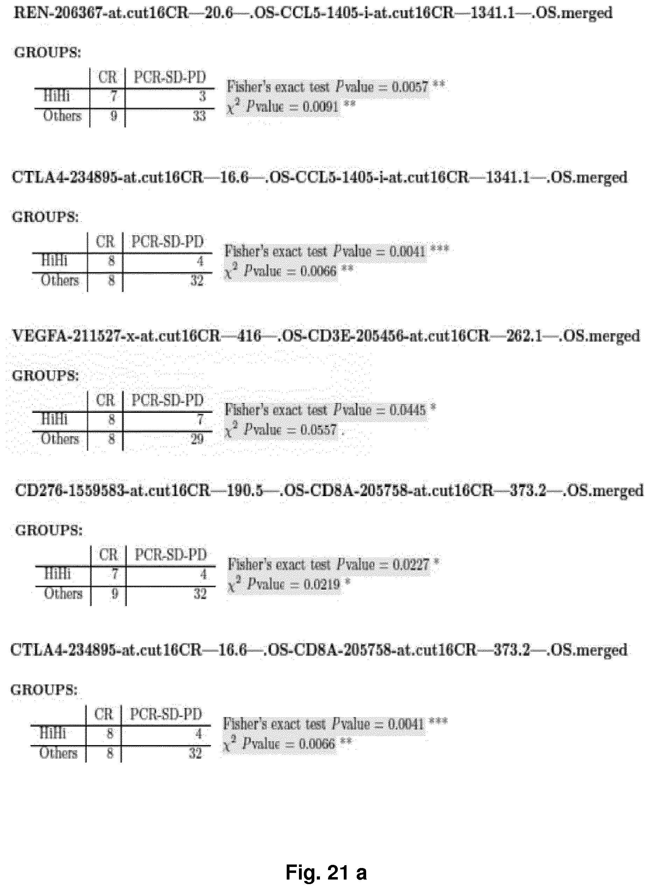

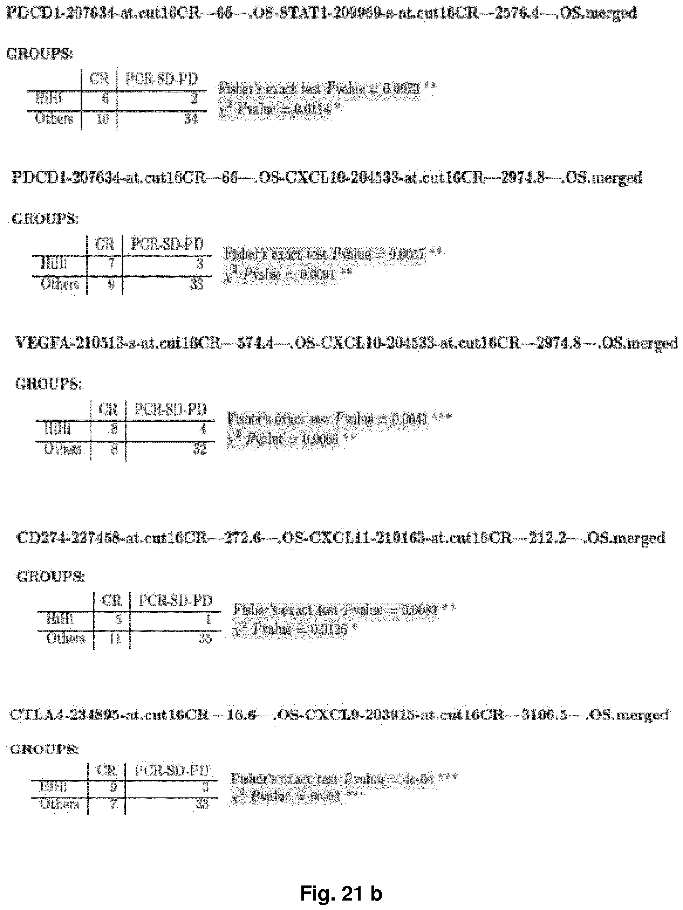

Figure 21 represents a Fisher-Exact-Test contingency table of late stage (III-IV) melanoma patients. All patients were submitted to chemotherapy. Four groups are represented, two subsets of patients with complete response (CR) and the two other subsets with no complete response (PR, SD, PD). Patients HiHi have a high expression of both genes compared to all other patients ("Others"). Representative examples of patients HiHi are illustrated such as: REN/CCL5, CTLA4/CCL5, VEGFA/CD3E, CD276/CD8A, CTLA4/CD8A, PDCD1/STAT1, PDCD1/CXCL10, VEGFA/CXCL10, CD274/CXCL11, CTLA4/CXCL9.

### EXAMPLE 1:

### 1. Determining whether a patient is a good responder or a bad responder for each of human adaptive immune response and human immunosuppressive response.

The expression level of genes CD3G (gene representative of the immune adaptive response) and of REN (gene representative of the immunosuppressive response) by a sample of colorectal tumour of a patient has been assessed as follows:
The patient had a Stage II colorectal cancer.

A tissue sample of his tumour was snap-frozen within 15 minutes after surgery and stored in liquid nitrogen. Total RNA of the tumour was isolated by homogenization with RNeasy isolation-kit (Qiagen, Valencia, CA). The integrity and the quantity of the RNA were evaluated on a bioanalyzer-2100 (Agilent Technologies, Palo Alto, CA). RT-PCR experiments were performed according to the manufacturer's instructions (Applied-Biosystems, Foster City, CA).

Quantitative real-time TaqMan-PCR was performed using Low-Density-Arrays and the 7900 robotic real-time PCR-system (Applied-Biosystems). 18S ribosomal RNA primers and probe were used as internal control.

Gene expression analyses were performed using Ct-values (threshold cycle) normalized to 18S ribosomal RNA (dCt).

Data were analyzed using the SDS Software v2.2 (Applied-Biosystems) and TME statistical module.

The results are as follows:

| | |
|---|---|
| CD3G: | dCt= 20.13 |
| REN: | dCt= 20.15 |

A dCt value lower than the relevant reference value means that the signal was detected earlier, i.e.: the expression of the gene is High.

A dCt value higher than the relevant reference value means that the signal was detected later, i.e.: the expression of the gene is Low.

The above results were compared with the following previously determined reference values: ELR_{CD3G} : 22.26 and ELR_{REN} : 21.48.

Since the dCt result for CD3G is lower than reference value: ELR_{CD3G}, the sample is considered as "high" as regards this criterion. Since the dCt result for REN is also lower than reference value: ELR_{REN}, the sample is considered as "high" as regards this criterion.

Therefore, the patient is considered as a good responder for human adaptive immune response and as a good responder for human immunosuppressive response.

### 2 Determining whether the patient would benefit of a treatment such as chemotherapy (standard of care for later stage patient (stage IV)) or anti-PDLl mAb treatment = whether the patient is a good responder to antitumoral treatment

Since the result of the comparison step consists of a "Hi" value for CD3G and a "Hi" value for REN, then an intermediate prognosis is provided and the contemplated treatment with chemotherapy or anti-PDLl mAb treatment or any other efficient treatment will likely provide a significant impact on the survival of the patient.

Therefore the patient should be treated and possible side effects of the treatment are justified by the benefit for the patient in terms of survival.

### EXAMPLE 2:

### 1. Determining whether a patient is a good responder or a bad responder for each of human adaptive immune response and human immunosuppressive response.

The expression level of genes CD3G and REN by a sample of colorectal tumour of a second patient has been assessed according to the procedure of example 1:
The patient had a Stage II colorectal cancer.

The results are as follows:

| | |
|---|---|
| CD3G: | dCt= 25.33 |
| REN: | dCt= 31.51 |

The above results where compared with the same determined reference values as above.

Since the result for CD3G is higher than reference value ELR_{CD3G}, the sample is considered as "Low" as regards this criterion. Since the result for REN is higher than reference value: ELR_{REN}, the sample is considered as "Low" as regards this criterion.

Therefore, the patient is considered as a bad responder for human adaptive immune response and as a bad responder for human immunosuppressive response.

### 2 Determining whether the patient would benefit of a treatment such as chemotherapy or anti-PDLl mAb treatment

Since the result of the comparison step consists of a "Lo" value for CD3G and a "Lo" value for REN, then a bad prognosis is provided and the contemplated antitumoral treatment with chemotherapy or anti-PDLl mAb treatment would provide no significant impact on the survival of the patient.

Therefore there is little or no therapeutic interest in treating the patient with chemotherapy or anti-PDLl mAb treatment because the treatment would have no valuable effects on the survival and would induce undesirable side effects.

### PREPARATION 1:

Reference values used for comparison consisting of "cut-off' values may for example be predetermined as described hereunder.

The RNA samples analyzed were from 108 different patients. These patients were used for gene expression experiments (Taqman cohort). The observation time in the cohorts was the interval between diagnosis and last contact (death or last follow-up). Data were censored at the last follow-up for patients without relapse, or death. The min:max values until progression/death or last follow-up were (0 to 136) months, respectively. Three patients for whom follow-up data were unavailable were excluded from survival analysis. Time to recurrence or disease-free time was defined as the interval from the date of surgery to confirmed tumor relapse date for relapsed patients and from the date of surgery to the date of last follow-up for disease-free patients.

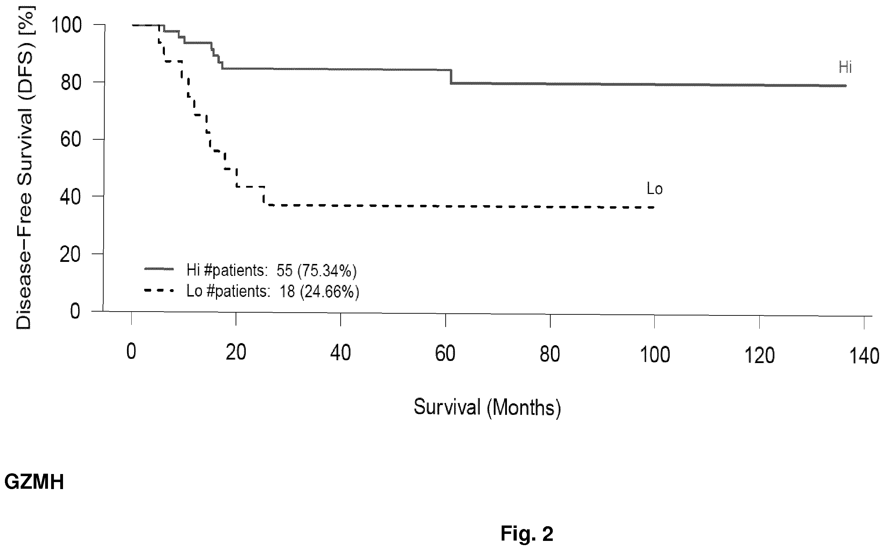

Histopathological and clinical findings were scored according to the UICC-TNM staging system. Post-surgical patient surveillance was performed at Laennec-HEGP Hospitals for all patients according to general practice for CRC patients. Adjuvant chemotherapy was administered or not to patients with stage II and III CRCs, and palliative chemotherapy to patients with advanced colorectal cancers (stage IV) and to patients without complete resection of the tumor. Adjuvant chemotherapy was fluorouracil (FU)-based. Follow-up data were collected prospectively and updated. A secure Web-based database, TME.db (Tumor MicroEnvironment Database), was built on a 3-tier architecture using Java-2 Enterprise-Edition (J2EE) to integrate the clinical data and the data from high-throughput technologies.
a) The records of colorectal cancer (CRC) patients who underwent a primary resection of their tumor at the Laennec-HEGP Hospitals (Paris - France) between 1996 and 2004 were reviewed. Frozen tumor samples available from Laennec-HEGP Hospitals from 1996-2004, with sufficient RNA quality and quantity, were selected (validation cohort 1, n=108);
b) The RNA samples from the 108 different patients were analyzed for determining the expression level of genes CD8A and CTLA4 for each tumour tissue sample of the collection, using the technique of Example 1 (Taqman cohort);
c) The tumour tissue samples have been ranked according to their expression level for each gene;
d) The 105 tumour tissue samples analysed have been classified in pairs of subsets of increasing, respectively decreasing, number of members ranked according to their expression level: Pair of subsets 1: The sample having the best expression level of gene CD8A on one side and all the 104 samples having a lower expression level of gene CD8A on the other side. Then Pair of subsets 2: the two samples having the best expression level of gene CD8A on one side and the 103 samples having a lower expression level of gene CD8A on the other side, etc... and finally pair of subsets 104: the 104 samples having the best expression level of gene CD8A on one side and the sample having the lowest expression level of gene CD8A on the other side.
e) For each tumour tissue sample, information relating to the actual clinical outcome for the corresponding patient (i.e. the duration of the disease-free survival (DFS) or the overall survival (OS) or both) has been obtained. The observation time in the cohorts was the interval between diagnosis and last contact (death or last follow-up). Data were censored at the last follow-up for patients without relapse, or death. The min.:max. values until progression/death or last follow-up were (0:136) months, respectively. Three patients for whom follow-up data were unavailable were excluded from survival analysis. Time to recurrence or disease-free time was defined as the interval from the date of surgery to confirmed tumor relapse date for relapsed patients and from the date of surgery to the date of last follow-up for disease-free patients;
f) For each pair of subsets, a Kaplan Meier curve has been drawn; typical Kaplan Meier curves from 73 different patients are shown on figures 1, 2 and 3;
g) For each pair of subsets the statistical significance (p value) between both subsets has been calculated and reported on a curve; a typical curve is shown on figure 2. The curve is generally concave and the extreme pairs of subsets have a high p value.
h) A reference value ELR is selected such as the p value is the smallest.

Figures 1, 2 and 3 represent Kaplan Meier curves (percentage of disease free survival versus time in months) concerning 73 pairs of subsets such as mentioned in preparation 1, for genes CD3G, GZMH and ITGAE and for the corresponding optimal reference value. Significance among patient groups was calculated using the log-rank test. P-values were corrected applying the method proposed by Altman et al (Altman DG, et al J Natl Cancer Inst 86:829-35, 1994). Hazard ratios (HR) were corrected as suggested by Holländer et al (Hollander N, et al Stat Med 23:1701-13, 2004).

Figure 1: CD3G. Curves for one subset of 60 members (highest expression level) and one subset of 13 members (lowest expression level) using dCt=20.13 as reference value for gene CD3G are shown. Kaplan-Meier curves for DFS illustrating the recurrence differences according to the immune gene expression, revealed a better prognosis (corrected HR=3.74, P<0.0005) associated with a high expression of CD3G within the tumor. The median survival was infinite (not reached) for patients with high expression of CD3G, whereas it was only 16 months for patients with low expression of CD3G.

Figure 2: GZMH. Curves for one subset of 55 members (highest expression level) and one subset of 18 members (lowest expression level) using dCt=22.17 as reference value for gene GZMH are shown. Kaplan-Meier curves for DFS illustrating the recurrence differences according to the immune gene expression, revealed a better prognosis (corrected HR=4.5, *P*<0.0005) associated with a high expression of GZMH within the tumor. The median survival was infinite (not reached) for patients with high expression of GZMH, whereas it was only 18 months for patients with low expression of GZMH.

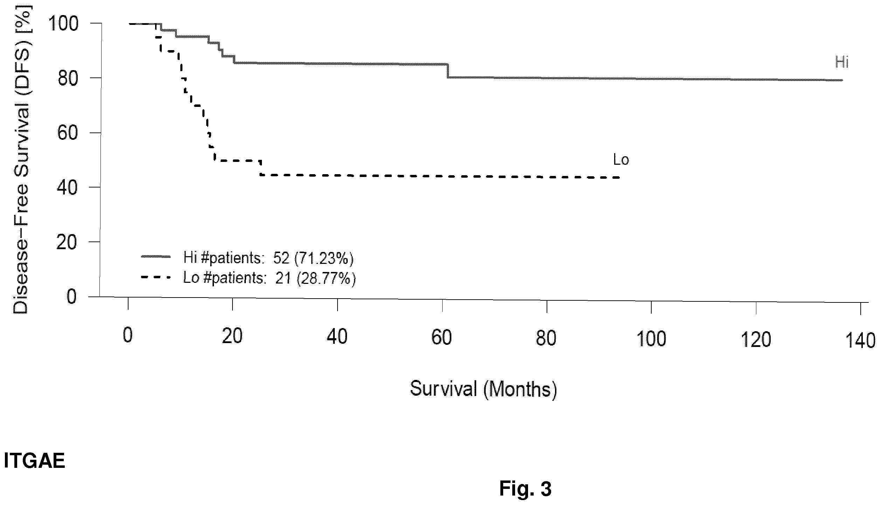

Figure 3: ITGAE. Curves for one subset of 52 members (highest expression level) and one subset of 21 members (lowest expression level) using dCt=21.89 as reference value for gene ITGAE are shown. Kaplan-Meier curves for DFS illustrating the recurrence differences according to the immune gene expression, revealed a better prognosis (corrected HR=4.21, *P*<0.0005) associated with a high expression of ITGAE within the tumor. The median survival was infinite (not reached) for patients with high expression of ITGAE, whereas it was only 16 months for patients with low expression of ITGAE.

Of particular note is the fact that for a same group of 73 patients, depending of the gene considered, the optimal reference value is obtained for different pairs of subsets.

On figures 4, 5 and 6, the smallest p value is obtained for the pair of subsets corresponding to an amount of about dCt=22.26 for CD3G, of about dCt=22.17 for GZMH and of about dCt=21.89 for ITGAE.

Significance among patient groups was calculated using the log-rank test. *P-*values and Hazard ratios (HR) were corrected like previously. The figure representing P-values plots as a function of the cut-points for gene expression revealed a range of significant cut-points (P<0.05), and a peak corresponding to the optimal cut-point, which can be further used as a reference value ELR.
Reference values ELR were obtained according to the above method for various combinations of genes and panels of 73 patients suffering of Stage II-III cancers and are reported in Table 4 hereunder. The first gene is representative of human adaptive immune response and the second gene is representative of human immunosuppressive response. In this table, each gene is followed by its corresponding reference value as obtained according to the above procedure.

**Table 4**

| | **Genes / dCt cutpoints** |
|---|---|
| 1 | CCL5 20.41 - REN 21.48 |
| 2 | CCR2 32.36 - IL17A 25.51 |
| 3 | CCR2 32.36 - REN 21.48 |
| 4 | CD247 21.28 - IL17A 25.51 |
| 5 | CD247 21.218 - REN 21.48 |
| 6 | CD3E 18.95 - REN 21.48 |
| 7 | CD3G 22.26 - IL17A 25.51 |
| 8 | CD3G 22.26 - REN 21.48 |
| 9 | CD8A 20.54 - IL17A 25.51 |
| 10 | CD8A 20.54 - REN 21.48 |
| 11 | CX3CL1 18.58 - CTLA4 20.20 |
| 12 | CX3CL1 18.58 - IHH 19.81 |
| 13 | GZMA 19.62 - PF4 20.02 |
| 14 | GZMA 19.62 - PROM1 23.16 |
| 15 | GZMA 19.62 - REN 21.48 |
| 16 | GZMA 19. 62 - TSLP 23.12 |
| 17 | GZMA 19. 62 - VEGF 15.44 |
| 18 | GZMB 20.24 - REN 21.48 |
| 19 | GZMH 22.17 - IL17A 25.51 |
| 20 | GZMH 22.17 - REN 21.48 |
| 21 | GZMK 22.57 - REN 21.48 |
| 22 | IFNG 25.03 - IL17A 25.50 |
| 23 | IL15 23.20 - CD274 23.02 |
| 24 | IL15 23.20 - CTLA4 20.20 |
| 25 | IL15 23.20 - IHH 19.81 |
| 26 | IL15 23.20 - TSLP 23.12 |
| 27 | IL15 23.20 - VEGF 15.44 |
| 28 | IRF1 15.49 - REN 21.48 |
| 29 | ITGAE 21.89 - IL17A 25.51 |
| 30 | ITGAE 21.89 - REN 21.48 |
| 31 | PRF1 21.17 - REN 21.48 |
| 32 | STAT1 16.51 - REN 21.48 |
| 33 | TBX21 23.47 - REN 21.48 |
| 34 | GZMK 22.57 - PDCD1 23.04 |
| 35 | CD247 21.28 - CD274 23.02 |
| 36 | PRF1 21.17 - PDCD1 23.04 |
| 37 | CCR2 32.36 - PF4 20.02 |
| 38 | CD247 21.28 - PDCD1 23.04 |
| 39 | CD3E 18.95 - PDCD1 23.04 |
| 40 | CCR2 32.36 - CD274 23.02 |
| 41 | CCR2 32.36 - PDCD1 23.04 |
| 42 | CCL5 20.41 - PDCD1 23.04 |
| 43 | CD3G 22.26 - PDCD1 23.04 |
| 44 | CD8A 20.54 - PDCD1 23.04 |
| 45 | TBX21 23.47 - PDCD1 23.04 |
| 46 | CD3G 22.26 - IHH 19.81 |
| 47 | CD3G 22.26 - PF4 20.02 |
| 48 | CD3G 22.26 - PROM1 23.16 |
| 49 | GZMB 20.24 - PDCD1 23.04 |
| 50 | GZMH 22.17 - PDCD1 23.04 |
| 51 | STAT1 16.51 - PDCD1 23.04 |
| 52 | STAT1 16.51 - PF4 20.02 |
| 53 | CD247 21.28 - PF4 20.02 |
| 54 | CD247 21.28 - CTLA4 20.20 |
| 55 | CD3G 22.26 - CTLA4 20.20 |
| 56 | CD8A 20.54 - CTLA4 20.20 |
| 57 | PRF1 21.17 - CTLA4 20.20 |
| 58 | CCL5 20.41 - CTLA4 20.20 |
| 59 | TBX21 23.47 - CTLA4 20.20 |
| 60 | STAT1 16.51 - IHH 19.81 |
| 61 | CD247 21.28 - IHH 19.81 |
| 62 | CD247 21.28 - PROM1 23.16 |
| 63 | PRF1 21.17 - PF4 20.02 |
| 64 | CD3G 22.26 - TSLP 23.12 |
| 65 | CCR2 32.36 - IHH 19.81 |
| 66 | CCR2 32.36 - CTLA4 20.20 |
| 67 | IFNG 25.03 - CD274 23.02 |
| 68 | GZMB 20.24 - CTLA4 20.20 |
| 69 | IFNG 25.03 - PDCD1 23.04 |
| 70 | CXCL11 20.78 - IL17A 25.51 |
| 71 | CXCL11 20.78 - REN 21.48 |

Table 5 hereunder summarises the results of a comparison for a same pair of genes between the following groups of patients:
- patients classified as "Hi Hi" and subjected to a chemotherapeutic treatment versus patients classified as "Hi Hi" but without chemotherapeutic treatment.
- patients not classified as "Hi Hi" (= Others) and subjected to a chemotherapeutic treatment versus patients not classified as "Hi Hi" and without chemotherapeutic treatment.

The results are expressed in logrank p value and corrected HR (hazard ratio) for disease free survival. Logrank is a well known test statistic which compares estimates of the hazard functions of the two groups at each observed event time. Logrank is constructed by computing the observed and expected number of events in one of the groups at each observed event time and then adding these to obtain an overall summary across all time points where there is an event.

Correction of the Hazard Ratio was made like previously according to Holländer.

**Table 5**

| | | CHIMIO-"HiHi" vs NO CHIMIO-"HiHi": DFS | | CHIMIO-Others vs NO CHIMIO-Others: DFS | |
|---|---|---|---|---|---|
| | **PAIRS OF GENES** | logrank P value | Corrected HR | logrank P value | Corrected HR |
| 1 | **CCL5 - REN** | 0,0000 | infinite | 0,0623 | 0,47 |
| 2 | **CCR2 - IL17A** | 0,0000 | infinite | 0,3115 | 1 |
| 3 | **CCR2 - REN** | 0,0000 | infinite | 0,1926 | 0,76 |
| 4 | **CD247 - IL17A** | 0,0000 | infinite | 0,3040 | 0,99 |
| 5 | **CD247 - REN** | 0,0000 | infinite | 0,1222 | 0,62 |
| 6 | **CD3E - REN** | 0,0000 | infinite | 0,0623 | 0,47 |
| 7 | **CD3G - IL17A** | 0,0000 | infinite | 0,3071 | 0,99 |
| 8 | **CD3G - REN** | 0,0000 | infinite | 0,1222 | 0,62 |
| 9 | **CD8A - IL17A** | 0,0000 | infinite | 0,1269 | 0,59 |
| 10 | **CD8A - REN** | 0,0000 | infinite | 0,0623 | 0,47 |
| 11 | **CX3CL1 - CTLA4** | 0,0000 | infinite | 0,0794 | 0,54 |
| 12 | **CX3CL1 - IHH** | 0,0000 | infinite | 0,1108 | 0,6 |
| 13 | **GZMA - PF4** | 0,0000 | infinite | 0,1747 | 0,72 |
| 14 | **GZMA - PROM1** | 0,0000 | infinite | 0,1671 | 0,71 |
| 15 | **GZMA - REN** | 0,0000 | infinite | 0,1229 | 0,63 |
| 16 | **GZMA - TSLP** | 0,0000 | infinite | 0,1796 | 0,73 |
| 17 | **GZMA - VEGF** | 0,0000 | infinite | 0,1458 | 0,67 |
| 18 | **GZMB - REN** | 0,0000 | infinite | 0,0623 | 0,47 |
| 19 | **GZMH - IL17A** | 0,0000 | infinite | 0,1130 | 0,57 |
| 20 | **GZMH - REN** | 0,0000 | infinite | 0,0623 | 0,47 |
| 21 | **GZMK-REN** | 0,0000 | infinite | 0,0855 | 0,52 |
| 22 | **IFNG - IL17A** | 0,0000 | infinite | 0,1999 | 0,76 |
| 23 | **IL15 - CD274** | 0,0000 | infinite | 0,1203 | 0,62 |
| 24 | **IL15 - CTLA4** | 0,0000 | infinite | 0,1632 | 0,7 |
| 25 | **IL15 - IHH** | 0,0000 | infinite | 0,1437 | 0,66 |
| 26 | **IL15 - TSLP** | 0,0000 | infinite | 0,0897 | 0,56 |
| 27 | **IL15 - VEGF** | 0,0000 | infinite | 0,0995 | 0,58 |
| 28 | **IRF1 - REN** | 0,0000 | infinite | 0,0623 | 0,47 |
| 29 | **ITGAE - IL17A** | 0,0000 | infinite | 0,1517 | 0,67 |
| 30 | **ITGAE - REN** | 0,0000 | infinite | 0,1222 | 0,62 |
| 31 | **PRF1 - REN** | 0,0000 | infinite | 0,0855 | 0,52 |
| 32 | **STAT1 - REN** | 0,0000 | infinite | 0,0855 | 0,52 |
| 33 | **TBX21 - REN** | 0,0000 | infinite | 0,0623 | 0,47 |
| 34 | **GZMK - PDCD1** | 0,0133 | 0,09 | 0,7191 | 0,01 |
| 35 | **CD247 - CD274** | 0,0152 | 0,07 | 0,8129 | 0,01 |
| 36 | **PRF1 - PDCD1** | 0,0152 | 0,14 | 0,8072 | 0 |
| 37 | **CCR2 - PF4** | 0,0185 | 1,06 | 0,8466 | 0 |
| 38 | **CD247 - PDCD1** | 0,0195 | 0,09 | 0,8878 | 0,01 |
| 39 | **CD3E - PDCD1** | 0,0207 | 0,1 | 0,8212 | 0 |
| 40 | **CCR2 - CD274** | 0,0208 | 0,07 | 0,9253 | 0,07 |
| 41 | **CCR2 - PDCD1** | 0,0220 | 0,09 | 0,8814 | 2,69 |
| 42 | **CCL5 - PDCD1** | 0,0225 | 0,09 | 0,9771 | 0,68 |
| 43 | **CD3G - PDCD1** | 0,0225 | 0,09 | 0,9771 | 0,68 |
| 44 | **CD8A - PDCD1** | 0,0225 | 0,09 | 0,9771 | 0,68 |
| 45 | **TBX21 - PDCD1** | 0,0225 | 0,09 | 0,9771 | 0,69 |
| 46 | **CD3G-IHH** | 0,0258 | 1,09 | 0,8280 | 0 |
| 47 | **CD3G - PF4** | 0,0258 | 1,11 | 0,8697 | 0,03 |
| 48 | **CD3G - PROM1** | 0,0305 | 0,09 | 0,6688 | 2,85 |
| 49 | **GZMB - PDCD1** | 0,0306 | 0,09 | 0,7839 | 3,13 |
| 50 | **GZMH - PDCD1** | 0,0348 | 0,09 | 0,9591 | 1,53 |
| 51 | **STAT1 - PDCD1** | 0,0356 | 0,09 | 0,9314 | 1,86 |
| 52 | **STAT1 - PF4** | 0,0423 | 1,31 | 0,9152 | 1,77 |
| 53 | **CD247 - PF4** | 0,0431 | 1,12 | 0,8630 | 2,64 |
| 54 | **CD247 - CTLA4** | 0,0433 | 0,14 | 0,5465 | 1,83 |
| 55 | **CD3G - CTLA4** | 0,0433 | 0,14 | 0,5465 | 1,83 |
| 56 | **CD8A - CTLA4** | 0,0433 | 0,14 | 0,5465 | 1,83 |
| 57 | **PRF1 - CTLA4** | 0,0433 | 0,14 | 0,5465 | 1,83 |
| 58 | **CCL5 - CTLA4** | 0,0433 | 0,14 | 0,5465 | 1,83 |
| 59 | **TBX21 - CTLA4** | 0,0433 | 0,14 | 0,5465 | 1,83 |
| 60 | **STAT1 - IHH** | 0,0438 | 1,32 | 0,9720 | 1,29 |
| 61 | **CD247 - IHH** | 0,0445 | 1,15 | 0,8675 | 2,51 |
| 62 | **CD247 - PROM1** | 0,0446 | 0,1 | 0,5521 | 1,9 |
| 63 | **PRF1 - PF4** | 0,0447 | 0,37 | 0,9258 | 1,55 |
| 64 | **CD3G - TSLP** | 0,0450 | 1,15 | 0,9300 | 1,93 |
| 65 | **CCR2 - IHH** | 0,0451 | 1,15 | 0,9204 | 2,11 |
| 66 | **CCR2 - CTLA4** | 0,0456 | 0,09 | 0,4968 | 1,6 |
| 67 | **IFNG - CD274** | 0,0463 | 0,06 | 0,3146 | 1,02 |
| 68 | **GZMB - CTLA4** | 0,0471 | 0,14 | 0,5195 | 1,7 |
| 69 | **IFNG - PDCD1** | 0,0472 | 0,14 | 0,4703 | 1,51 |
| 70 | **CXCL11 - IL17A** | 0,0444 | 0,37 | 0,2397 | 1,37 |
| 71 | **CXCL11 - REN** | 0,0259 | 0,32 | 0,7328 | 6,34 |

Results obtained with other pairs of genes such as CD3G-VEGF, CD3E-VEGF and CD8A-VEGF are provided hereafter.

A logrank P value below 0.05 is significative.

A corrected hazard ratio whose value is infinite reveals the absence of tumour recurrence. The gap between the two curves is infinite and the P-value is very significative.

Conversely, a corrected Hazard Ratio whose value is 1 or close to 1 reveals that the curves of both subsets of patients are superimposed. Therefore since one curve represents treated patients and the other curve represents untreated patients, one concludes that the treatment was ineffective.

### ANALYSIS OF THE RESULTS

For example, for the first pair of genes (CCL5 - REN)
- The logrank P value of 0.0000 (<0.0001), therefore below 0.05 is significative and the value of the corrected Hazard Ratio is infinite which means that for the group of patients having high expression levels for both genes there is no tumour recurrence in the group of treated patients and a chemotherapeutic treatment provided an important improvement to the condition of the patients.
- On the other side, for the three groups of patients who do not have high expression levels for both genes, the value of the corrected hazard ratio is 0.47, close to 1 because the curves are close to each other (typical curves of this kind are illustrated in figure 7 - three bottom curves), and a non-significant P value. Therefore, for patients who do not have high expression levels for both genes, a chemotherapeutic treatment provided no improvement to the condition of the patients.

CONCLUSION: Therefore, the results of Table 5 show that a reference value for a expression level for both genes of all the pairs of genes allow determining whether a chemotherapeutic treatment will provide an improvement to the condition of a treated patient. These combinations of genes, following the procedure, are therefore predictive markers of response to treatment.

Figure 7 represents a Kaplan Meier curve for DFS for four subsets of cancer stage II/III patients. Patients of a pair of subsets (CHIMIO) were submitted to chemotherapies and patients of a second pair of subsets (NON = no chemotherapy) were not treated. Each pair comprises a subset of "Hi-Hi" patients and the other pair is constituted by the three other groups of patients (Others).

Legend of the curves:
Solid line: "Hi-Hi" treated patients (14 patients)
Dotted line: "Hi-Hi" untreated patients (25 patients)
Dashed line: treated "other" patients (12 patients)
Line constituted of alternance of dots and lines: untreated "other" patients (22 patients)

Analysis of the curves shows that:
- The logrank P value of 0.0000 (<0.0001), therefore below 0.05 is significative and the value of the corrected hazard ratio is infinite which means that no patients "HiHi" that received chemotherapy had tumor recurrence for 120 months.
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have similar survival than treated or untreated "Others". In contrast, treated HiHi patients have prolonged survival. The survival is considerably enhanced in the group consisting of "Hi-Hi" treated patients (responders to antitumoral treatment) in comparison with any other group of patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Absolute reference values are quite specific of a given method for assessing the level of expression of a gene. For comparison purposes of course the same units should preferably be used. When the technique used for determining expression levels of genes implies an exponential phenomenon such as quantitative PCR, a corrected reference value should preferably be used. In the present experiments, dCt normalized values compared to housekeeping gene (18S) were used.

Figure 8 represents a Kaplan Meier curve for OS for four subsets of cancer stage IV patients, of the kind of the curve of Figure 7.

The legend of the curves is the same as in Figure 7:
Solid line: "Hi-Hi" treated patients (9 patients)
Dotted line: "Hi-Hi" untreated patients (9 patients)
Dashed line: treated "other" patients (8 patients)
Line constituted of alternance of dots and lines: untreated "other" patients (3 patients)

Analysis of the curves shows that:
- The group of patients having high expression levels for both genes has the best survival, but in the group of chemotherapy-treated patients there was no improvement to the condition of the patients, as compared to non-treated patients.
- On the other side, for the two groups of patients who do not have high expression levels for both genes, the non-treated patients had the worst outcome (50% recurrence at 6 months), whereas chemotherapy-treated patients had prolonged disease-free survival (50% recurrence at 20 months), showing improvement to the condition of these patients after treatment.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Absolute reference values are quite specific of a given method for assessing the level of expression of a gene. For comparison purposes of course the same units should preferably be used. When the technique used for determining expression levels of genes implies an exponential phenomenon such as quantitative PCR, a corrected reference value should preferably be used. In the present experiments, dCt normalized values compared to housekeeping gene (18S) were used.

Figure 9 illustrates some combinations of genes of both types. White dots represent genes representative of an adaptative immune response whereas black dots represent genes representative of an immunosuppressive response. The lines between genes of both types represent significant correlations of gene combinations. Thick lines are the most significant. A gene comprising many connecting lines is more universal than a gene comprising only a few lines or a single line. For example, gene CD3G representative of an adaptative immune response is linked by thick lines to five different genes representative of an immunosuppressive response. Therefore, gene CD3G is a quite universal gene representative of an adaptative immune response. On the other side, PDCD1 is linked by numerous thick lines to different genes representative of an adaptative immune response. Therefore, gene PDCD1 is a quite universal gene representative of an immunosuppressive response.

Figure 10 represents a Kaplan Meier curve for four subsets of colorectal cancer stage I/III patients. Patients of a pair of subsets (CHIMIO) were submitted to chemotherapies and patients of a second pair of subsets (NON = no chemotherapy) were not treated. Each pair comprises a subset of "Hi-Hi" patients and the other pair is constituted by the three other groups of patients (Others).

Legend of the curves:
Solid line: "Hi-Hi" treated patients (3 patients)
Dotted line: "Hi-Hi" untreated patients (11 patients)
Dashed line: treated "other" patients (24 patients)
Line constituted of alternance of dots and lines: untreated "other" patients (58 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have similar survival than treated or untreated "Others". In contrast, treated HiHi patients have prolonged survival. The survival is considerably enhanced in the group consisting of "Hi-Hi" treated patients (responders to antitumoral treatment) in comparison with any other group of patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Absolute reference values are quite specific of a given method for assessing the level of expression of a gene. For comparison purposes of course the same units should preferably be used. When the technique used for determining expression levels of genes implies an exponential phenomenon such as quantitative PCR, a corrected reference value should preferably be used. In the present experiments, dCt normalized values compared to housekeeping gene (18S) were used.

Figure 11 represents a Kaplan Meier curve for four subsets of colorectal cancer stage II/III patients. Patients of a pair of subsets (CHIMIO) were submitted to chemotherapies and patients of a second pair of subsets (NON = no chemotherapy) were not treated. Each pair comprises a subset of "Hi-Hi" patients and the other pair is constituted by the three other groups of patients (Others).

Legend of the curves:
Solid line: "Hi-Hi" treated patients (15 patients)
Dotted line: "Hi-Hi" untreated patients (20 patients)
Dashed line: treated "other" patients (11 patients)
Line constituted of alternance of dots and lines: untreated "other" patients (28 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have similar survival than treated or untreated "Others". In contrast, treated HiHi patients have prolonged survival. The survival is considerably enhanced in the group consisting of "Hi-Hi" treated patients (responders to antitumoral treatment) in comparison with any other group of patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Absolute reference values are quite specific of a given method for assessing the level of expression of a gene. For comparison purposes of course the same units should preferably be used. When the technique used for determining expression levels of genes implies an exponential phenomenon such as quantitative PCR, a corrected reference value should preferably be used. In the present experiments, dCt normalized values compared to housekeeping gene (18S) were used.

Figure 12 represents a Kaplan Meier curve for four subsets of colorectal cancer stage II/III patients. Patients of a pair of subsets (CHIMIO) were submitted to chemotherapies and patients of a second pair of subsets (NON = no chemotherapy) were not treated. Each pair comprises a subset of "Hi-Hi" patients and the other pair is constituted by the three other groups of patients (Others).

Legend of the curves:
Solid line: "Hi-Hi" treated patients (14 patients)
Dotted line: "Hi-Hi" untreated patients (22 patients)
Dashed line: treated "other" patients (12 patients)
Line constituted of alternance of dots and lines: untreated "other" patients (26 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have similar survival than treated or untreated "Others". In contrast, treated HiHi patients have prolonged survival. The survival is considerably enhanced in the group consisting of "Hi-Hi" treated patients (responders to antitumoral treatment) in comparison with any other group of patients.

In the group of HiHi patients the hazard ratio for DFS between untreated and treated patients is 6.36 (p<0.05) and the hazard ratio for OS between untreated and treated patients is 4.81 (p<0.05).

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Absolute reference values are quite specific of a given method for assessing the level of expression of a gene. For comparison purposes of course the same units should preferably be used. When the technique used for determining expression levels of genes implies an exponential phenomenon such as quantitative PCR, a corrected reference value should preferably be used. In the present experiments, dCt normalized values compared to housekeeping gene (18S) were used.

Figure 13 represents a Kaplan Meier curve for four subsets of colorectal cancer stage I/III patients. Patients of a pair of subsets (CHIMIO) were submitted to chemotherapies and patients of a second pair of subsets (NON = no chemotherapy) were not treated. Each pair comprises a subset of "Hi-Hi" patients and the other pair is constituted by the three other groups of patients (Others).

Legend of the curves:
Solid line: "Hi-Hi" treated patients (3 patients)
Dotted line: "Hi-Hi" untreated patients (12 patients)
Dashed line: treated "other" patients (24 patients)
Line constituted of alternance of dots and lines: untreated "other" patients (57 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have similar survival than treated or untreated "Others". In contrast, treated HiHi patients have prolonged survival. The survival is considerably enhanced in the group consisting of "Hi-Hi" treated patients (responders to antitumoral treatment) in comparison with any other group of patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Absolute reference values are quite specific of a given method for assessing the level of expression of a gene. For comparison purposes of course the same units should preferably be used. When the technique used for determining expression levels of genes implies an exponential phenomenon such as quantitative PCR, a corrected reference value should preferably be used. In the present experiments, dCt normalized values compared to housekeeping gene (18S) were used.

Figure 14 represents a Kaplan Meier curve for four subsets of lung cancer early stage (I/II) patients. Patients of a pair of subsets (ACT) were submitted to chemotherapies and patients of a second pair of subsets (OBS = no chemotherapy) were not treated. Each pair comprises a subset of "Hi-Hi" patients and the other pair is constituted by the three other groups of patients (Others).

Legend of the curves:
Solid line: "Hi-Hi" treated patients (7 patients)
Dotted line: "Hi-Hi" untreated patients (4 patients)
Dashed line: treated "other" patients (43 patients)
Line constituted of alternance of dots and lines: untreated "other" patients (36 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have poor prognosis compared to treated or untreated "Others". In contrast, the group consisting of "Hi-Hi" treated patients (responders to antitumoral treatment) have a better survival than untreated HiHi patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Figure 15 represents a Kaplan Meier curve for four subsets of lung cancer early stage (I/II) patients. Patients of a pair of subsets (ACT) were submitted to chemotherapies and patients of a second pair of subsets (OBS = no chemotherapy) were not treated. Each pair comprises a subset of "Hi-Hi" patients and the other pair is constituted by the three other groups of patients (Others).

Legend of the curves:
Solid line: "Hi-Hi" treated patients (3 patients)
Dotted line: "Hi-Hi" untreated patients (4 patients)
Dashed line: treated "other" patients (46 patients)
Line constituted of alternance of dots and lines: untreated "other" patients (37 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have similar survival than treated or untreated "Others". In contrast, treated HiHi patients have prolonged survival. The survival is considerably enhanced in the group consisting of "Hi-Hi" treated patients (responders to antitumoral treatment) in comparison with any other group of patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Figure 16 represents a Kaplan Meier curve for four subsets of lung cancer early stage (I/II) patients. Patients of a pair of subsets (ACT) were submitted to chemotherapies and patients of a second pair of subsets (OBS = no chemotherapy) were not treated. Each pair comprises a subset of "Hi-Hi" patients and the other pair is constituted by the three other groups of patients (Others).

Legend of the curves:
Solid line: "Hi-Hi" treated patients (11 patients)
Dotted line: "Hi-Hi" untreated patients (7 patients)
Dashed line: treated "other" patients (39 patients)
Line constituted of alternance of dots and lines: untreated "other" patients (33 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have poor prognosis compared to treated or untreated "Others". In contrast, the group consisting of "Hi-Hi" treated patients (responders to antitumoral treatment) have a better survival than untreated HiHi patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Figure 17 represents a Kaplan Meier curve for four subsets of lung cancer early stage (I/II) patients. Patients of a pair of subsets (ACT) were submitted to chemotherapies and patients of a second pair of subsets (OBS = no chemotherapy) were not treated. Each pair comprises a subset of "Hi-Hi" patients and the other pair is constituted by the three other groups of patients (Others).

Legend of the curves:
Solid line: "Hi-Hi" treated patients (39 patients)
Dotted line: "Hi-Hi" untreated patients (33 patients)
Dashed line: treated "other" patients (11 patients)
Line constituted of alternance of dots and lines: untreated "other" patients (7 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have similar survival than treated or untreated "Others". In contrast, treated HiHi patients have prolonged survival. The survival is considerably enhanced in the group consisting of "Hi-Hi" treated patients (responders to antitumoral treatment) in comparison with any other group of patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Figure 18 represents a Kaplan Meier curve for four subsets of lung cancer early stage (I/II) patients. Patients of a pair of subsets (ACT) were submitted to chemotherapies and patients of a second pair of subsets (OBS = no chemotherapy) were not treated. Each pair comprises a subset of "Hi-Hi" patients and the other pair is constituted by the three other groups of patients (Others).

Legend of the curves:
Solid line: "Hi-Hi" treated patients (9 patients)
Dotted line: "Hi-Hi" untreated patients (7 patients)
Dashed line: treated "other" patients (41 patients)
Line constituted of alternance of dots and lines: untreated "other" patients (33 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have poor prognosis compared to treated or untreated "Others". In contrast, the group consisting of "Hi-Hi" treated patients (responders to antitumoral treatment) have a better survival than untreated HiHi patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Figure 19 represents a Kaplan Meier curve for OS for four subsets of advanced ovarian cancer patients. Patients were submitted to chemotherapies and four subsets of patients were categorized. Patients with complete response (CR) and with no complete response (IR). Each pair comprises a subset of "Hi-Hi" patients and the other pair is constituted by the three other groups of patients (Others).

Legend of the curves:
Solid line: CR "Hi-Hi" patients (15 patients)
Dotted line: IR "Hi-Hi" patients (8 patients)
Dashed line: CR"other" patients (3 patients)
Line constituted of alternance of dots and lines: IR "other" patients (2 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have similar survival than treated or untreated "Others". In contrast, treated HiHi patients have prolonged survival. The survival is considerably enhanced in the group consisting of "Hi-Hi" treated patients (responders to antitumoral treatment) in comparison with any other group of patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Figure 20 represents a Kaplan Meier curve for OS for four subsets of advanced ovarian cancer patients. Patients were submitted to chemotherapies and four subsets of patients were categorized. Patients with complete response (CR) and with no complete response (IR). Each pair comprises a subset of "Hi-Hi" patients and the other pair is constituted by the three other groups of patients (Others).

Legend of the curves:
Solid line: CR "Hi-Hi" patients (16 patients)
Dotted line: IR "Hi-Hi" patients (7 patients)
Dashed line: CR "other" patients (2 patients)
Line constituted of alternance of dots and lines: IR "other" patients (3 patients)

Analysis of the curves shows that:
- The curves of treated or untreated "other" patients are similar. Therefore the antitumoral treatment provided no significant beneficial effect to patients belonging to the group of non-"Hi-Hi" patients.
- The curves of treated or untreated "HiHi" patients are different. Untreated HiHi patients have similar survival than treated or untreated "Others". In contrast, treated HiHi patients have prolonged survival. The survival is considerably enhanced in the group consisting of "Hi-Hi" treated patients (responders to antitumoral treatment) in comparison with any other group of patients.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

Figure 21 represents a Fisher-Exact-Test contingency table of late stage (III-IV) melanoma patients. All patients were submitted to chemotherapy. Four groups are represented, two subsets of patients with complete response (CR) and the two other subsets with no complete response (PR, SD, PD). Patients HiHi have a high expression of both genes compared to all other patients ("Others"). Representative examples of patients HiHi are illustrated such as: REN/CCL5, CTLA4/CCL5, VEGFA/CD3E, CD276/CD8A, CTLA4/CD8A, PDCD1/STAT1, PDCD1/CXCL10, VEGFA/CXCL10, CD274/CXCL11, CTLA4/CXCL9. Significant Fisher Test illustrates the fact that patients with gene combinations HiHi vs Others have different disease outcome and different response to treatment. Patients with HiHi have an increased frequency of complete response to treatment.

Accordingly, the comparison of expression levels of one or several genes representative of human adaptive immune response and the expression level of one or several genes representative of human immunosuppressive response to predetermined reference values is a reliable criterion for determining whether a patient could be successfully treated against cancer or not, in the sense that the risk-benefit balance is favourable to the patient. This illustrates the value of stratifying patients with such combination of genes to predict their response to treatment.

The continuum of cancer immunosurveillance: prognostic, predictive and mechanistic signatures, Galon J*, Angell HK, Bedognetti D, and Marincola F, Immunity. 2013 July 39(1), 11-26 further shows that a good association between the results obtained by the methods of the present invention and favorable disease outcome.

### REFERENCES:

Throughout this application, various references describe the state of the art to which this invention pertains.

## Claims

1. An *in vitro* method for assessing the responsiveness of a patient suffering from solid cancer to antitumoral treatment comprising the steps of:
i) determining in a tumor sample obtained from a patient, an expression level of at least one gene representative of human adaptive immune response and an expression level of at least one gene representative of human immunosuppressive response,
ii) comparing the expression of said at least one gene representative of human adaptive immune response and said at least one gene representative of human immunosuppressive response determined at step i) with predetermined reference values selected such as said predetermined reference values separate a panel of patients with a cancer into two groupings according to the expression level of said genes and to survival of patients according to Kaplan Meier curves analyses and associated logrank p values;
iii) concluding whether the patient has a good, when the level is higher than the predetermined reference value, or a bad, when the level is lower than the predetermined reference value, adaptive immune response and a good or a bad immunosuppressive response
iv) determining whether the patient will advantageously receive an antitumoral treatment in view of said good or bad adaptative immune and immunosuppressive responses
wherein the at least one gene representative of human adaptive immune response is selected from the group consisting of
| |
|---|
| CCL5 |
| CCR2 |
| CD247 |
| CD3E |
| CD3G |
| CD8A |
| CX3CL1 |
| CXCL11 |
| GZMA |
| GZMB |
| GZMH |
| GZMK |
| IFNG |
| IL15 |
| IRF1 |
| ITGAE |
| PRF1 |
| STAT1 |
| TBX21 |
and said at least one gene representative of human immunosuppressive response is selected from the group consisting of
| |
|---|
| CD274 |
| CTLA4 |
| IHH |
| IL17A |
| PDCD1 |
| PF4 |
| PROM1 |
| REN |
| TSLP |
| VEGFA |

2. The method according to claim 1, wherein a single gene representative of the adaptive immune response and a single gene representative of the immunosuppressive response are used in step i).

3. The method according to one of claims 1 or 2, wherein a pair of gene representative of human adaptive immune response and of gene representative of human immunosuppressive response is selected from the group consisting of:
| |
|---|
| CCL5 - REN |
| CCR2 - IL17A |
| CCR2 - REN |
| CD247 - IL17A |
| CD247 - REN |
| CD3E - REN |
| CD3G - IL17A |
| CD3G - REN |
| CD8A - IL17A |
| CD8A - REN |
| CX3CL1 - CTLA4 |
| CX3CL1 - IHH |
| GZMA - PF4 |
| GZMA - PROM1 |
| GZMA - REN |
| GZMA - TSLP |
| GZMA - VEGFA |
| GZMB - REN |
| GZMH - IL17A |
| GZMH - REN |
| GZMK - REN |
| IFNG - IL17A |
| IL15 - CD274 |
| IL15 - CTLA4 |
| IL15 - IHH |
| IL15 - TSLP |
| IL15 - VEGFA |
| IRF1 - REN |
| ITGAE - IL17A |
| ITGAE - REN |
| PRF1 - REN |
| STAT1 - REN |
| TBX21 - REN |
| GZMK - PDCD1 |
| CD247 - CD274 |
| PRF1 - PDCD1 |
| CCR2 - PF4 |
| CD247 - PDCD1 |
| CD3E - PDCD1 |
| CCR2 - CD274 |
| CCR2 - PDCD1 |
| CCL5 - PDCD1 |
| CD3G - PDCD1 |
| CD8A - PDCD1 |
| TBX21 - PDCD1 |
| CD3G - IHH |
| CD3G - PF4 |
| CD3G - PROM1 |
| GZMB - PDCD1 |
| GZMH - PDCD1 |
| STAT1 - PDCD1 |
| STAT1 - PF4 |
| CD247 - PF4 |
| CD247 - CTLA4 |
| CD3G - CTLA4 |
| CD8A - CTLA4 |
| PRF1 - CTLA4 |
| CCL5 - CTLA4 |
| TBX21 - CTLA4 |
| STAT1 - IHH |
| CD247 - IHH |
| CD247 - PROM1 |
| PRF1 - PF4 |
| CD3G - TSLP |
| CCR2 - IHH |
| CCR2 - CTLA4 |
| IFNG - CD274 |
| GZMB - CTLA4 |
| IFNG - PDCD1 |
| CXCL11 - IL17A |
| CXCL11 - REN |

4. The method according to any one of claims 1 to 3, wherein said gene of the human immunosuppressive response is CD274.

5. The method according to any one of claims 1 to 4, wherein said solid cancer is selected from the group consisting of adrenal cortical cancer, anal cancer, bile duct cancer, bladder cancer, bone cancer, brain and central nervous system cancer, breast cancer, cervical cancer, colorectal cancer, endometrial cancer, esophagus cancer, gallbladder cancer, gastrointestinal carcinoid tumors, Kaposi's sarcoma, kidney cancer, laryngeal and hypopharyngeal cancer, liver cancer, lung cancer, mesothelioma, nasal cavity and paranasal sinus cancer, nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma, salivary gland cancer, skin cancer, stomach cancer, testicular cancer, thymus cancer, thyroid cancer, vaginal cancer, vulvar cancer, and uterine cancer.

6. The method according to claim 5, wherein said cancer is a colorectal cancer.

7. The method according to claim 5, wherein said cancer is a lung cancer.

8. The method according to claim 5, wherein said cancer is an ovarian cancer.

9. The method according to claim 5, wherein said cancer is a skin cancer, said skin cancer being melanoma.

10. Use of a kit for implementing the step of determination of an expression level of at least one gene representative of human adaptive immune response and the expression level of at least one gene representative of human immunosuppressive response of the method of one of claims 1-9 wherein said kit comprises means for specifically measuring the expression levels of said at least one gene of the adaptive immune response and of said at least one gene of the immunosuppressive response.

11. The use of a kit according to claim 10, wherein said means consist in probes, primers and antibodies specific for said at least one gene of the adaptive immune response and of said at least one gene of the immunosuppressive response.

12. A chemotherapeutic agent, an immunotherapeutic agent or a radiotherapeutic agent for use in the treatment of a cancer patient who is considered as responder to antitumoral treatment according to the method of one of claims 1 to 9.

## Patentansprüche

1. *In vitro* Verfahren zum Beurteilen der Empfindlichkeit eines Patienten, der an einem soliden Krebs leidet, gegenüber einer Antitumorbehandlung, umfassend die Schritte:
i) Bestimmen eines Expressionsspiegels von mindestens einem Gen, das für die menschliche adaptive Immunantwort repräsentativ ist, und eines Expressionsspiegels von mindestens einem Gen, das für die menschliche immunsuppressive Antwort repräsentativ ist, in einer Tumorprobe, die von einem Patienten erhalten wurde,
ii) Vergleichen der in Schritt i) bestimmten Expression des mindestens einen Gens, das für die menschliche adaptive Immunantwort repräsentativ ist, und des mindestens einen Gens, das für die menschliche immunsuppressive Antwort repräsentativ ist, mit vorbestimmten Referenzwerten, die so ausgewählt sind, dass die vorbestimmten Referenzwerte ein Panel an Patienten mit einem Krebs in zwei Gruppierungen teilt entsprechend des Expressionsspiegels der Gene und des Überlebens der Patienten entsprechend Kaplan Meier Kurvenanalysen und zugehörigen Logrank p-Werten;
iii) Folgern, ob der Patient eine gute, wenn der Spiegel höher als der vorbestimmte Referenzwert ist, oder eine schlechte, wenn der Spiegel niedriger als der vorbestimmte Referenzwert ist, adaptive Immunantwort und eine gute oder eine schlechte immunsuppressive Antwort aufweist
iv) Bestimmen, ob der Patient vorteilhafterweise eine Antitumorbehandlung in Anbetracht der guten oder schlechten adaptiven Immunantwort und immunsuppressiven Antwort erhalten wird
wobei das mindestens eine Gen, das für die menschliche adaptive Immunantwort repräsentativ ist, ausgewählt ist aus der Gruppe bestehend aus
| |
|---|
| CCL5 |
| CCR2 |
| CD247 |
| CD3E |
| CD3G |
| CD8A |
| CX3CL1 |
| CXCL11 |
| GZMA |
| GZMB |
| GZMH |
| GZMK |
| IFNG |
| IL15 |
| IRF1 |
| ITGAE |
| |
| PRF1 |
| STAT1 |
| TBX21 |
und das mindestens eine Gen, das für die menschliche immunsuppressive Antwort repräsentativ ist, ausgewählt ist aus der Gruppe bestehend aus
| |
|---|
| CD274 |
| CTLA4 |
| IHH |
| IL17A |
| PDCD1 |
| PF4 |
| PROM1 |
| REN |
| TSLP |
| VEGFA |

2. Verfahren nach Anspruch 1, wobei ein einzelnes Gen, das für die adaptive Immunantwort repräsentativ ist, und ein einzelnes Gen, das für die immunsuppressive Antwort repräsentativ ist, in Schritt i) verwendet werden.

3. Verfahren nach einem der Ansprüche 1 oder 2, wobei ein Genpaar, das für die menschliche adaptive Immunantwort repräsentativ ist, und ein Genpaar, das für die menschliche immunsuppressive Antwort repräsentativ ist, ausgewählt ist aus der Gruppe bestehend aus:
| |
|---|
| CCL5 - REN |
| CCR2 - IL17A |
| CCR2 - REN |
| CD247 - IL17A |
| CD247 - REN |
| CD3E - REN |
| CD3G - IL17A |
| CD3G - REN |
| CD8A - IL17A |
| CD8A - REN |
| CX3CL1 - CTLA4 |
| CX3CL1 - IHH |
| GZMA - PF4 |
| GZMA - PROM1 |
| GZMA - REN |
| GZMA - TSLP |
| GZMA - VEGFA |
| GZMB - REN |
| GZMH - IL17A |
| GZMH - REN |
| GZMK - REN |
| IFNG - IL17A |
| IL15 - CD274 |
| IL15 - CTLA4 |
| IL15 - IHH |
| IL15 - TSLP |
| IL15 - VEGFA |
| IRF1 - REN |
| ITGAE - IL17A |
| ITGAE - REN |
| PRF1 - REN |
| STAT1 - REN |
| TBX21 - REN |
| GZMK - PDCD1 |
| CD247 - CD274 |
| PRF1 - PDCD1 |
| CCR2 - PF4 |
| CD247 - PDCD1 |
| CD3E - PDCD1 |
| CCR2 - CD274 |
| CCR2 - PDCD1 |
| CCL5 - PDCD1 |
| CD3G - PDCD1 |
| CD8A - PDCD1 |
| TBX21 - PDCD1 |
| CD3G - IHH |
| CD3G - PF4 |
| CD3G - PROM1 |
| GZMB - PDCD1 |
| GZMH - PDCD1 |
| STAT1 - PDCD1 |
| STAT1 - PF4 |
| CD247 - PF4 |
| CD247 - CTLA4 |
| CD3G - CTLA4 |
| CD8A - CTLA4 |
| PRF1 - CTLA4 |
| CCL5 - CTLA4 |
| TBX21 - CTLA4 |
| STAT1 - IHH |
| CD247 - IHH |
| CD247 - PROM1 |
| PRF1 - PF4 |
| CD3G - TSLP |
| CCR2 - IHR |
| CCR2 - CTLA4 |
| IFNG - CD274 |
| GZMB - CTLA4 |
| IFNG - PDCD1 |
| CXCL11 - IL17A |
| CXCL11 - REN |

4. Verfahren nach einem der Ansprüche 1 bis 3, wobei das Gen der menschlichen immunsuppressiven Antwort CD274 ist.

5. Verfahren nach einem der Ansprüche 1 bis 4, wobei der solide Krebs ausgewählt ist aus der Gruppe bestehend aus Nebennierenrindenkrebs, Analkrebs, Gallengangkrebs, Blasenkrebs, Knochenkrebs, Gehirntumor und Krebs des zentralen Nervensystems, Brustkrebs, Gebärmutterhalskrebs, Darmkrebs, Endometriumkarzinom, Speiseröhrenkrebs, Gallenblasenkrebs, gastrointestinale Karzinoidtumore, Kaposi Sarkom, Nierenkrebs, Kehlkopf- und Hypopharynxkrebs, Leberkrebs, Lungenkrebs, Mesotheliom, Nasenhöhlen- und Nasennebenhöhlenkrebs, Nasopharynxkrebs, Neuroblastom, Mundhöhlen- und Oropharynxkrebs, Eierstockkrebs, Bauchspeicheldrüsenkrebs, Peniskrebs, Hypophysenkrebs, Prostatakrebs, Retinoblastom, Rhabdomyosarkom, Speicheldrüsenkrebs, Hautkrebs, Magenkrebs, Hodenkrebs, Thymuskrebs, Schilddrüsenkrebs, Vaginalkrebs, Vulvakrebs und Gebärmutterkrebs.

6. Verfahren nach Anspruch 5, wobei der Krebs ein Darmkrebs ist.

7. Verfahren nach Anspruch 5, wobei der Krebs ein Lungenkrebs ist.

8. Verfahren nach Anspruch 5, wobei der Krebs ein Eierstockkrebs ist.

9. Verfahren nach Anspruch 5, wobei der Krebs ein Hautkrebs ist, wobei der Hautkrebs ein Melanom ist.

10. Verwendung eines Kits zum Umsetzen des Schritts der Bestimmung eines Expressionsspiegels von mindestens einem Gen, das für die menschliche adaptive Immunantwort repräsentativ ist, und des Expressionsspiegels von mindestens einem Gen, das für die menschliche immunsuppressive Antwort repräsentativ ist, des Verfahrens nach einem der Ansprüche 1 - 9, wobei das Kit Mittel umfasst für ein spezifisches Messen der Expressionsspiegel des mindestens einen Gens der adaptiven Immunantwort und des mindestens einen Gens der immunsuppressiven Antwort.

11. Verwendung eines Kits nach Anspruch 10, wobei das Mittel in Sonden, Primern und Antikörpern besteht, die für das mindestens eine Gen der adaptiven Immunantwort und des mindestens einen Gens der immunsuppressiven Antwort spezifisch sind.

12. Chemotherapeutisches Mittel, immuntherapeutisches Mittel oder radiotherapeutisches Mittel zur Verwendung bei der Behandlung eines Krebspatienten, der als ein Responder auf eine Antitumorbehandlung nach dem Verfahren nach einem der Ansprüche 1 bis 9 erachtet wird.

## Revendications

1. Procédé *in vitro* pour évaluer la réponse d'un patient souffrant d'un cancer solide à un traitement antitumoral comprenant les étapes de :
i) déterminer dans un échantillon de tumeur obtenu d'un patient, d'un niveau d'expression d'au moins un gène représentatif de la réponse immunitaire adaptative humaine et d'un niveau d'expression d'au moins un gène représentatif de la réponse immunosuppressive humaine,
ii) comparer l'expression dudit au moins un gène représentatif de la réponse immunitaire adaptative humaine et dudit au moins un gène représentatif de la réponse immunosuppressive humaine déterminée à l'étape i) à des valeurs de référence prédéterminées sélectionnées de manière à ce que que lesdites valeurs de référence prédéterminées séparent un panel de patients ayant un cancer en deux groupements selon le niveau d'expression desdits gènes et la survie des patients selon des analyses de courbes de Kaplan Meier et des valeurs p de logrank associées ;
iii) établir si le patient a une bonne, lorsque le niveau est supérieur à la valeur de référence prédéterminée, ou mauvaise, lorsque le niveau est inférieur à la valeur de référence prédéterminée, réponse immunitaire adaptative et une bonne ou mauvaise réponse immunosuppressive,
iv) déterminer si le patient recevra de façon avantageuse ou non un traitement antitumoral au vu desdites bonnes ou mauvaises réponses immunitaire adaptative et immunosuppressive
dans lequel l'au moins un gène représentatif de la réponse immunitaire adaptative humaine est sélectionné dans le groupe constitué de
| |
|---|
| CCL5 |
| CCR2 |
| CD247 |
| CD3E |
| CD3G |
| CD8A |
| CX3CL1 |
| CXCL11 |
| GZMA |
| GZMB |
| GZMH |
| GZMK |
| IFNG |
| IL15 |
| IRF1 |
| ITGAE |
| PRF1 |
| STAT1 |
| TBX21 |
et ledit au moins un gène représentatif de la réponse immunosuppressive humaine est sélectionné dans le groupe constitué de
| |
|---|
| CD274 |
| CTLA4 |
| IHH |
| IL17A |
| PDCD1 |
| PF4 |
| PROM1 |
| REN |
| TSLP |
| VEGFA |

2. Procédé selon la revendication 1, dans lequel un seul gène représentatif de la réponse immunitaire adaptative et un seul gène représentatif de la réponse immunosuppressive sont utilisés à l'étape i).

3. Procédé selon l'une des revendications 1 ou 2, dans lequel une paire d'un gène représentatif de la réponse immunitaire adaptative humaine et d'un gène représentatif de la réponse immunosuppressive humaine est sélectionnée dans le groupe constitué de :
| |
|---|
| CCL5 - REN |
| CCR2 - IL17A |
| CCR2 - REN |
| CD247 - IL17A |
| CD247 - REN |
| CD3E - REN |
| CD3G - IL17A |
| CD3G - REN |
| CD8A - IL17A |
| CD8A - REN |
| CX3CL1 - CTLA4 |
| CX3CL1 - IHH |
| GZMA - PF4 |
| GZMA - PROM1 |
| GZMA - REN |
| GZMA - TSLP |
| GZMA - VEGFA |
| GZMB - REN |
| GZMH - IL17A |
| GZMH - REN |
| GZMK - REN |
| IFNG - IL17A |
| IL15 - CD274 |
| IL15 - CTLA4 |
| IL15 - IHH |
| IL15 - TSLP |
| IL15 - VEGFA |
| IRF1 - REN |
| ITGAE - IL17A |
| ITGAE - REN |
| PRF1 - REN |
| STAT1 - REN |
| TBX21 - REN |
| GZMK - PDCD 1 |
| CD247 - CD274 |
| PRF1 - PDCD1 |
| CCR2 - PF4 |
| CD247 - PDCD1 |
| CD3E - PDCD1 |
| CCR2 - CD274 |
| CCR2 - PDCD1 |
| CCL5 - PDCD1 |
| CD3G - PDCD1 |
| CD8A - PDCD1 |
| TBX21 - PDCD1 |
| CD3G - IHH |
| CD3G - PF4 |
| CD3G - PROM1 |
| GZMB - PDCD1 |
| GZMH - PDCD1 |
| STAT1 - PDCD1 |
| STAT1 - PF4 |
| CD247 - PF4 |
| CD247 - CTLA4 |
| CD3G - CTLA4 |
| CD8A - CTLA4 |
| PRF1 - CTLA4 |
| CCL5 - CTLA4 |
| TBX21 - CTLA4 |
| STAT1 - IHH |
| CD247 - IHH |
| CD247 - PROM1 |
| PRF1 - PF4 |
| CD3G - TSLP |
| CCR2 - IHH |
| CCR2-CTLA4 |
| IFNG - CD274 |
| GZMB - CTLA4 |
| IFNG - PDCD1 |
| CXCL11 - IL17A |
| CXCL11 - REN |

4. Procédé selon l'une quelconque des revendications 1 à 3, dans lequel ledit gène de la réponse immunosuppressive humaine est CD274.

5. Procédé selon l'une quelconque des revendications 1 à 4, dans lequel ledit cancer solide est sélectionné dans le groupe constitué d'un cancer de la corticosurrénale, d'un cancer anal, d'un cancer des voies biliaires, d'un cancer de la vessie, d'un cancer des os, d'un cancer du système nerveux central et du cerveau, d'un cancer du sein, d'un cancer du col de l'utérus, d'un cancer colorectal, d'un cancer de l'endomètre, d'un cancer de l'oesophage, d'un cancer de la vésicule biliaire, de tumeurs carcinoïdes gastro-intestinales, d'un sarcome de Kaposi, d'un cancer du rein, d'un cancer du larynx et de l'hypopharynx, d'un cancer du foie, d'un cancer du poumon, d'un mésothéliome, d'un cancer de la cavité nasale et des sinus paranasaux, d'un cancer nasopharyngé, d'un neuroblastome, d'un cancer de la cavité buccale et de l'oropharynx, d'un cancer des ovaires, d'un cancer du pancréas, d'un cancer du pénis, d'un cancer pituitaire, d'un cancer de la prostate, d'un rétinoblastome, d'un rhabdomyosarcome, d'un cancer des glandes salivaires, d'un cancer de la peau, d'un cancer de l'estomac, d'un cancer des testicules, d'un cancer du thymus, d'un cancer de la thyroïde, d'un cancer du vagin, d'un cancer de la vulve, et d'un cancer de l'utérus.

6. Procédé selon la revendication 5, dans lequel ledit cancer est un cancer colorectal.

7. Procédé selon la revendication 5, dans lequel ledit cancer est un cancer du poumon.

8. Procédé selon la revendication 5, dans lequel ledit cancer est un cancer des ovaires.

9. Procédé selon la revendication 5, dans lequel ledit cancer est un cancer de la peau, ledit cancer de la peau étant un mélanome.

10. Utilisation d'un kit pour mettre en oeuvre l'étape de détermination d'un niveau d'expression d'au moins un gène représentatif de la réponse immunitaire adaptative humaine et du niveau d'expression d'au moins un gène représentatif de la réponse immunosuppressive humaine du procédé selon l'une des revendications 1 à 9 dans laquelle ledit kit comprend des moyens pour spécifiquement mesurer les niveaux d'expression dudit au moins un gène de la réponse immunitaire adaptative et dudit au moins un gène de la réponse immunosuppressive.

11. Utilisation d'un kit selon la revendication 10, dans lequel lesdits moyens consistent en des sondes, des amorces et des anticorps spécifiques dudit au moins un gène de la réponse immunitaire adaptative et dudit au moins un gène de la réponse immunosuppressive.

12. Agent chimiothérapeutique, agent immunothérapeutique ou agent radiothérapeutique pour son utilisation dans le traitement d'un patient atteint d'un cancer qui est considéré comme répondeur à un traitement antitumoral selon le procédé de l'une quelconque des revendications 1 à 9.
